# EUROPÄISCHE PATENTSCHRIFT

(11) **EP 0 760 818 B1**
(45) Veröffentlichungstag und Bekanntmachung des Hinweises auf die Patenterteilung: **06.03.2002**
(21) Anmeldenummer: 95921745.6
(22) Anmeldetag: 11.05.1995
(51) Int. Cl.: C07D 475/04, C07D 475/06, A61K 31/505

(54) **VERWENDUNG VON TETRAHYDROPTERIDIN-DERIVATEN ALS HEMMSTOFFE DER NO-SYNTHASE**
USE OF TETRAHYDROPTERIDINE DERIVATIVES AS NO-SYNTHASE INHIBITORS
UTILISATION DE DERIVES DE TETRAHYDROPTERIDINE COMME INHIBITEURS DE LA NO-SYNTHASE

(30) Priorität: 24.05.1994 DE 4418097
(43) Veröffentlichungstag der Anmeldung: 12.03.1997
(73) Patentinhaber: HOECHST AKTIENGESELLSCHAFT, 65929 Frankfurt am Main (DE)
(72) Erfinder: PFLEIDERER, Wolfgang, D-78464 Konstanz (DE); SCHMIDT, Harald, D-97337 Dettelbach (DE); HENNING, Rainer, Shibuya-ku Tokyo 151 (JP)
(74) Vertreter: Viering, Jentschura & Partner
(86) Internationale Anmeldenummer: EP9501785
(87) Internationale Veröffentlichungsnummer: WO9532203

(56) Entgegenhaltungen:
- WO-A-94/14780
- CHEM. BER., 1970, 722-34, KONRAD, GUENTHER ET AL 'Pteridines. XXXVII. Synthesis and properties of 2,4-diaminopteridines and their 5,6,7,8-tetrahydro derivatives'
- BRITISH JOURNAL OF PHARMACOLOGY, Bd. 107, Nr. 4, 1992 Seiten 1088-1091, JORENS, P.G. ET AL 'Pterins inhibit nitric oxide synthase activity in rat alveolar macrophages' in der Anmeldung erwähnt
- MOLECULAR PHARMACOLOGY, Bd. 43, Nr. 1, 1993 Seiten 6-10, SAKAI, N. ET AL 'Tetrahydrobiopterin is required for cytokine-induced nitric oxide production in a murine macrophage cell line (RAW 264)'
- BIOCHEMICAL AND BIOPHYSICAL RESEARCH COMMUNICATIONS, Bd. 199, Nr. 2, 15.März 1994 Seiten 504-510, SCHOEDON, G. ET AL 'Nitric oxide production depends on preceding tetrahydrobiopterin synthesis by endothelial cells: selective suppression of induced nitric oxide production by sepiapterin reductase inhibitors'
- AMERICAN JOURNAL OF PHYSIOLOGY, Bd. 266, Nr. 4, April 1994 Seiten L455-L460, NAKAYAMA, D.K. ET AL 'Tetrahydrobiopterin synthesis and inducible nitric oxide production in pulmonary artery smooth muscle'
- JOURNAL OF EXPERIMENTAL MEDICINE, Bd. 172, Dezember 1990 Seiten 1599-1607, WERNER-FELMAYER, G. ET AL 'Tetrahydrobiopterin-dependent formation of nitrite and nitrate in murine fibroblasts'

## Beschreibung

Die vorliegende Erfindung betrifft Pteridin-Derivate der allgemeinen Formel I, 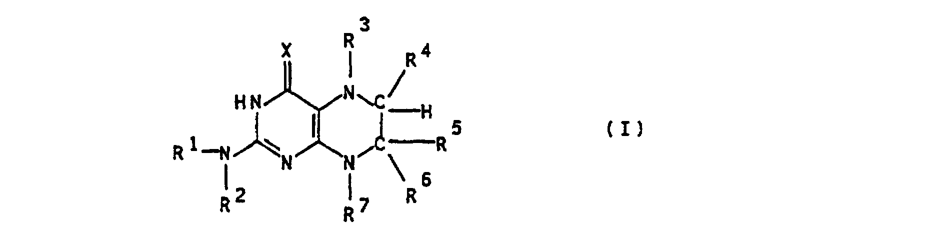 die aufgrund ihrer Fähigkeit zur Modulation der körpereigenen Stickstoffmonoxid-Produktion wertvolle Arzneimittel zur Vorbeugung und Bekämpfung von Krankheitszuständen sind, die durch einen gestörten Stickstoffmonoxid-Spiegel gekennzeichnet sind.

Stickstoffmonoxid (NO) spielt in verschiedensten physiologischen Prozessen eine bedeutende Rolle (siehe z.B. R.Henning, Nachr. Chem. Tech. Lab. 41 (1993), 413; H.H.H.W.Schmidt et al., Biochim. Biophys. Acta 1178 (1993), 153).

Es wirkt z.B. relaxierend auf die glatte Gefäßmuskulatur und ist auf diese Weise maßgeblich an der Regulierung des Blutdrucks beteiligt, es steuert über eine Hemmung der Thrombocytenaggregation die Blutgerinnung, und es ist beispielsweise im Gehirn als Neurotransmitter in den Aufbau des Langzeitgedächtnisses involviert. In den NANC-Nerven des peripheren Nervensystems fungiert NO ebenfalls als Botenstoff. Die zelltoxische Wirkung des NO wird von Macrophagen für die Infektionsabwehr ausgenutzt.

Endogenes NO wird mit Hilfe mindestens drei verschiedener NO-Synthase-Isoenzyme aus Arginin gebildet (siehe z.B. J.F.Kerwin, Jr., M.Heller, Med. Res. Rev. 14 (1994), 23). Sie unterscheiden sich bezüglich ihrer Lokalisation im Organismus, ihrer Regulierbarkeit durch Ca²⁺/Calmodulin sowie ihrer Induzierbarkeit durch Endotoxine und Cytokine. Die konstitutiven, calciumabhängigen NO-Synthasen finden sich beispielsweise in Endothel (Typ III) und im Gehirn (Typ I) und sind dort in die Regulation von Blutdruck und -gerinnung bzw. in Reizleitungsprozesse involviert. Die cytokin-induzierbare, calciumunabhängige Isoform (Typ II) tritt in Macrophagen, Glattmuskelzellen und und Hepatocyten auf. Sie ist in der Lage, über einen langen Zeitraum relativ große Mengen NO zu produzieren und wird für entzündliche Prozesse und die zelltoxische Aktivität der Macrophagen verantwortlich gemacht.

Ein gestörter NO-Haushalt hat schwerwiegende Erkrankungen und Schädigungen zur Folge. So führt die exzessive Bildung von NO im septischen oder hämorrhagischen Schock zu massiven pathologischen Blutdruckabfällen. Übersteigerte NO-Produktion ist an der Entstehung von Typ-1-Diabetes und Atherosklerose beteiligt und scheint auch für die glutamatinduzierte Neurotoxizität nach cerebraler Ischämie verantwortlich zu sein. Hohe NO-Konzentrationen können darüberhinaus durch Desaminierung von Cytosin zu DNA-Schädigungen führen. Beispiele für Erkrankungen, die durch einen Mangel an endogenem NO mittelbar oder unmittelbar hervorgerufen werden, sind arterieller Bluthochdruck, Störungen der Hämostase, koronare Herzkrankheit und die erektile Dysfunktion.

Der Ansatz, eine Modulation der NO-Produktion zur Behandlung dieser Krankheitsbilder zu verwenden, wurde bislang nur mit Hilfe von Arginin-Analoga realisiert (GB-A-2240041; WO-A-93/13055). Als weitere potentielle NO-Synthase-Hemmstoffe werden in der Literatur N-Iminoethylornithin (McCall et al., Br.J.Pharmacol. 102 (1991), 234), Aminoguanidin (T.P.Misko et al, Eur.J.Pharmacol. 233 (1993), 119; BP-A-547588) und 7-Nitroindazol (P.K.Moore et al, Br.J.Pharmacol. 108 (1993), 296) diskutiert.

Verschiedene Pteridin-Derivate kommen in der Natur vor, und auch Verwendungen von Pteridin-Derivaten als Pharma-Wirkstoffe sind beschrieben. So betrifft die EP-B-108 890 den Einsatz von Pteridin-Derivaten zur Behandlung von Krankheiten, die auf einen Katecholamin-Mangel zurückgehen, wie etwa der Parkinson'schen Krankheit, und die Anwendung von Pteridinen zur Behandlung der Phenylketonurie (siehe auch E.C.Bigham et al., Chemistry and Biology of Pteridines (1986), S.111, Walter de Gruyter & Co., Berlin, New York); dazu gehören auch bestimmte Verbindungen der allgemeinen Formel I, in der X für Sauerstoff steht und R¹, R², R³ und R⁷ gleichzeitig für Wasserstoff stehen. Die Wirkung des natürlich vorkommenden 5,6,7,8-Tetrahydrobiopterins und von Analoga dieser Substanz auf die NO-Produktion wurde z.B. von Kwon et al. (J.Biol.Chem. 264 (1989), 20496) oder Giovanelli et al. (Proc.Natl.Acad.Sci. USA 88 (1991), 7091) untersucht. Danach stimuliert Tetrahydrobiopterin die NO-Produktion und ist ein Cofaktor der NO-Synthasen. Eine Stimulation der NO-Produktion wurde auch für das 7,8-Dihydrobiopterin, gefunden. Nach Overfeld et al.(Br.J.Pharmacol.107 (1992), 1008) kann 5,6,7,8-Tetrahydrobiopterin aber auch hemmend auf die NO-Produktion wirken. Der Effekt ist konzentrationsabhängig.

Über eine Erhöhung der NO-Synthase-Aktivität durch 6-Methyl-5,6,7,8-tetrahydropterin berichten Hevel und Marletta (Biochemistry 31 (1992), 7160). Von Giovanelli et al. (loc. cit.) wurde diese nur bei höheren Konzentrationen beobachtet. Verschiedene andere Pterin-Derivate zeigten in diesen Untersuchungen keine signifikanten Effekte.

Überraschend wurde nun gefunden, daß Pteridin-Derivate der allgemeinen Formel I insbesondere hemmend die endogene NO-Produktion modulieren und somit als Arzneimittel bei Krankheiten geeignet sind, die durch einen überhöhten NO-Spiegel charakterisiert sind.

Gegenstand der vorliegenden Erfindung ist die Verwendung von Pteridin-Derivaten der allgemeinen Formel I, 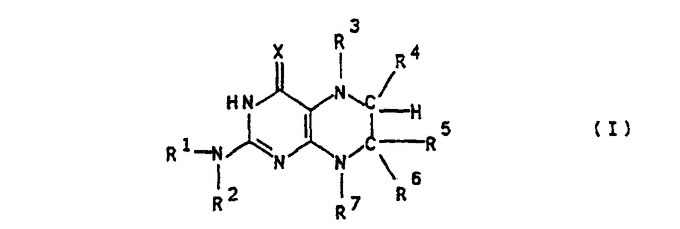 in der
- X: für O oder NH steht;
- R¹: für Wasserstoff, Methyl, (C₁-C₅)-Alkanoyl, Nicotinoyl oder (1-Methyl-3-pyridinio)carbonyl steht;
- R²: für Wasserstoff oder Methyl steht;
- R³: für Wasserstoff, Methyl, Ethyl, Benzyl, (C₁-C₅)-Alkanoyl, unsubstituiertes Benzoyl, substituiertes Benzoyl, Pyridoyl, Thienylcarbonyl, einen der Reste 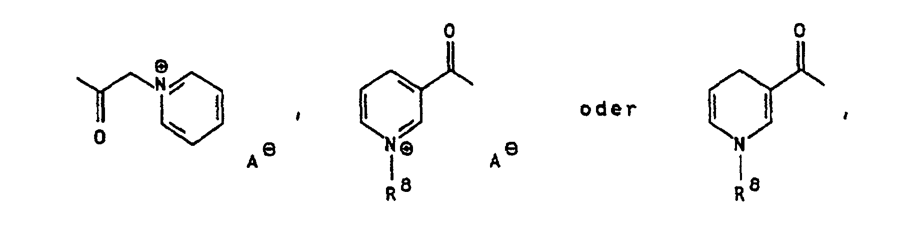 den Rest R⁹R^{9a}N-CO-, den Rest R⁹R^{9a}N-CS-, Phenoxycarbonyl oder Benzyloxycarbonyl steht;
- R⁴: für Wasserstoff, (C₂-C₅)-Alkyl, unsubstituiertes Phenyl, substituiertes Phenyl oder den Rest R^{4a}-CH₂-steht;
- R^{4a}: für Wasserstoff, (C₁-C₄)-Alkylmercapto, den Rest -S(O)ₘR¹⁰, wobei m für die Zahlen 1 oder 2 steht, den Rest -NR¹¹R¹² oder den Rest -OR¹³ steht oder
- R³ und R^{4a}: zusammen für die Gruppierung -CO-O- stehen, wobei deren Carbonyl-C-Atom an die 5-Position des Pteridinmoleküls gebunden ist;
- R⁵: für Wasserstoff, Methyl oder Phenyl steht;
- R⁶: für Wasserstoff oder Methyl steht;
- R7: für Wasserstoff oder Methyl steht;
- R⁸: für (C₁-C₁₀)-Alkyl oder Benzyl steht;
- R⁹: für Wasserstoff, (C₁-C₆)-Alkyl, Cyclohexyl, Phenyl oder Benzoyl steht;
- R^{9a}: für Wasserstoff, Methyl oder Ethyl steht;
- R¹⁰: für Methyl steht:
- R¹¹ und R¹²: unabhängig voneinander für Wasserstoff oder Methyl stehen;
- R¹³: für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
- A^{θ}: für ein pharmakologisch verträgliches Anion steht;
und ihrer tautomeren Formen sowie ihrer pharmakologisch verträglichen Salze zur Herstellung eines Medikaments zur Vorbeugung und Behandlung von Krankheiten, die durch einen erhöhen Stickstoffmonoxid-Spiegel bedingt sind.

Alkylgruppen können geradkettig oder verzweigt sein. Dies gilt auch, wenn sie in anderen Gruppen, beispielsweise in Alkoxy-, Alkylmercapto-, Alkoxycarbonyl- oder Alkanoylgruppen auftreten. Beispiele für Alkylgruppen, die in den erfindungsgemäß zu verwendenden Verbindungen der allgemeinen Formel als solche, also als (C₁-C₄)-, (C₂-C₅)-, (C₁-C₆)- oder (C₁-C₁₀)-Alkyl, oder in anderen Gruppen auftreten können, sind Methyl, Ethyl, n-Propyl, i-Propyl-, n-Butyl, i-Butyl, sek-Butyl, tert-Butyl, n-Pentyl, 3-Methylbutyl, 2,2-Dimethylpropyl, n-Hexyl, n-Heptyl, n-Octyl, n-Nonyl oder n-Decyl. Beispiele speziell für (C₁-C₅)-Alkanoyl sind Formyl, Acetyl, Propionyl, n-Butyryl, i-Butyryl, n-Valeroyl, 3-Methyl-n-butyryl oder 2,2-Dimethylpropionyl. Beispiele für (C₁-C₂)-Alkoxysind Methoxy und Ethoxy. Beispiele für unsubstituierte oder im Alkylteil durch einen Phenylrest substituiertes 2-Amino-(C₂-C₆)-alkanoyl sind die entsprechenden Reste der Aminosäuren Glycin, Alanin, Valin, Leucin, Isoleucin, Norvalin, Norleucin, Phenylglycin oder Phenylalanin.

Substituierte Phenylgruppen und Benzoylgruppen können ein- oder mehrfach substituiert sein, bevorzugt sind sie ein- bis dreifach substituiert. Die Substituenten können sich in beliebigen Positionen befinden, ein einzelner Substituent z.B. in der ortho-, meta- oder para-Position. Bei Mehrfachsubstitution können die Substituenten gleich oder verschieden sein. Als Substituenten können beispielsweise vorliegen (C₁-C₄)-Alkyl, (C₁-C₄)-Alkoxy, Amino, (C₁-C₄)-Alkylamino, Di-((C₁-C₄)-alkyl)amino, (C₁-C₅)-Alkanoylamino, Nitro oder Halogen, wobei nur bis zu zwei Nitrogruppen zugegen sein können. Halogen ist beispielsweise Fluor, Chlor, Brom oder Iod.

Pyridoyl kann 2-, 3- oder 4-Pyridoyl sein, wobei 3-Pyridoyl (= Nicotinoyl) bevorzugt ist. Thienylcarbonyl kann 2- oder 3-Thienylcarbonyl sein.

Beispiele für Säuren der Formel HA, deren Anion in den erfindungsgemäß zu verwendenden Verbindungen der allgemeinen Formel I vorliegen kann, sind Chlorwasserstoff, Bromwasserstoff, Iodwasserstoff, Schwefelsäure-, Phosphorsäure, Salpetersäure, Methansulfonsäure, Toluolsulfonsäure, Essigsäure, Propionsäure, Benzoesäure, Milchsäure, Weinsäure, Citronensäure, Maleinsäure, Fumarsäure. A^{θ} kann aber auch für ein Anion einer anderen unter pharmakologischen und pharmazeutischen Aspekten geeigneten anorganischen oder organischen Säuren stehen und kann, wenn die Verbindung der allgemeinen Formel I in Form eines Säureadditionssalzes vorliegt, mit dem durch diese Salzbildung eingebrachten Anion übereinstimmen oder sich davon unterscheiden.

Die Verbindungen der allgemeinen Formel I können in verschiedenen tautomeren Formen und in verschiedenen stereoisomeren Formen vorliegen. Die vorliegende Erfindung umfaßt nicht nur die Verwendung aller tautomeren Formen, sondern auch die aller stereoisomeren Formen, also z.B. die von reinen Enantiomeren, von Enantiomerengemischen und Racematen, von reinen Diastereomeren und Diastereomerengemischen oder von cis/trans-Isomeren.

X steht bevorzugt für Sauerstoff.

Bevorzugt stehen R¹ und R² gleichzeitig für Wasserstoff oder gleichzeitig für Methyl, oder es steht R² für Wasserstoff und gleichzeitig R¹ für (C₁-C₅)-Alkanoyl, insbesondere Acetyl, i-Butyryl oder Pivaloyl, oder für Nicotinoyl oder (1-Methyl-3-pyridinio)carbonyl. Besonders bevorzugt stehen R¹ und R² gleichzeitig für Wasserstoff oder R² für Wasserstoff und gleichzeitig R¹ für i-Butyryl.

R³ steht bevorzugt für Wasserstoff oder einen Acylrest oder einen (Thio-)Carbamoylrest. Unter Acylresten sind dabei bevorzugt (C₁-C₅)-Alkanoyl, insbesondere Formyl, Acetyl und Pivaloyl, unsubstituiertes oder ein-, zweioder dreifach substituiertes Benzoyl, Thienylcarbonyl, sowie Nicotinoyl und die Reste 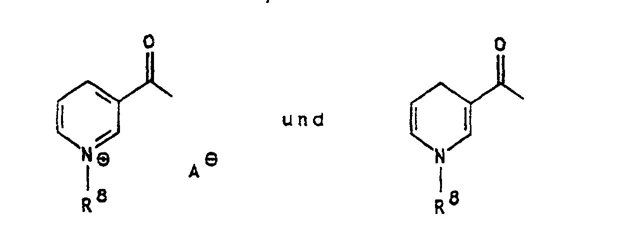 zu verstehen, wobei R⁸ bevorzugt für Methyl, n-Octyl oder Benzyl steht und bevorzugte Substituenten in substituierten Benzoylgruppen Halogen und Methoxy sind. In den Carbamoylresten R⁹R^{9a}N-CO- steht R⁹ bevorzugt für Wasserstoff, (C₁-C₄)-Alkyl, insbesondere tert-Butyl, sowie Cyclohexyl und Phenyl und gleichzeitig R^{9a} für Wasserstoff, oder es stehen R⁹ und R^{9a} gleichzeitig für Methyl. In den Thiocarbamoylresten R⁹R^{9a}N-CS- steht R⁹ bevorzugt für Wasserstoff, (C₁-C₄)-Alkyl, insbesondere Methyl und Ethyl, sowie für Phenyl und Benzoyl und gleichzeitig R^{9a} für Wasserstoff. Besonders bevorzugt steht R³ für Wasserstoff, den N-Phenylthiocarbamoylrest, den unsubstituierten Benzoylrest, den Nicotinoylrest oder einen von letzterem durch Alkylierung oder Benzylierung am Nicotinoylstickstoff abgeleiteten 3-Pyridinocarbonylrest oder einen in der 1-Position des Pyridinrings alkylierten oder benzylierten 1,4-Dihydro-3-pyridylcarbonylrest.

R⁴ steht bevorzugt für Wasserstoff, Phenyl, durch eine Acetylaminogruppe, insbesondere durch eine para-ständige Acetylaminogruppe substituiertes Phenyl oder den Rest R^{4a}-CH₂-.

R^{4a} steht bevorzugt für Wasserstoff oder den Rest -OR¹³. Steht R⁴ für Wasserstoff oder steht R^{4a} für Wasserstoff, so ist es besonders bevorzugt, wenn R³ gleichzeitig für einen Acylrest oder einen (Thio-)Carbamoylrest steht.

R⁵ steht bevorzugt für Wasserstoff.

R⁷ steht bevorzugt für Wasserstoff.

R^{9a} steht bevorzugt für Wasserstoff.

R¹³ steht bevorzugt für Wasserstoff, (C₁-C₁₀)-Alkyl oder einen Acylrest, insbesondere (C₁-C₅)-Alkanoyl oder unsubstituiertes oder im Alkylteil durch einen Phenylrest substituiertes 2-Amino-(C₂-C₆)-alkanoyl. Besonders bevorzugt steht R¹³ für (C₁-C₁₀)-Alkyl oder (C₁-C₅)-Alkanoyl, speziell Acetyl. Steht R¹³ für (C₁-C₁₀)-Alkyl, so sind n-Alkylreste sowie i-Propyl, i-Butyl und tert-Butyl bevorzugt.

Bevorzugt ist die Verwendung von Verbindungen der allgemeinen Formel I, in der einer oder mehrere Stubstituenten bevorzugte Bedeutungen haben. Bevorzugt ist weiterhin die Verwendung von Pteridin-Derivaten der allgemeinen Formel Ia, 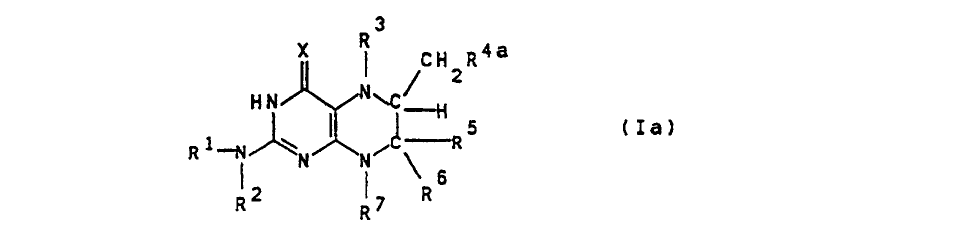 in der
- X: für O oder NH steht;
- R¹: für Wasserstoff, Methyl, (C₁-C₅)-Alkanoyl, Nicotinoyl oder (1-Methyl-3-pyridinio)carbonyl steht;
- R²: für Wasserstoff oder Methyl steht:
- R³: für Wasserstoff, Methyl, Ethyl, Benzyl, (C₁-C₅)-Alkanoyl, Benzoyl, Nicotinoyl, einen der Reste 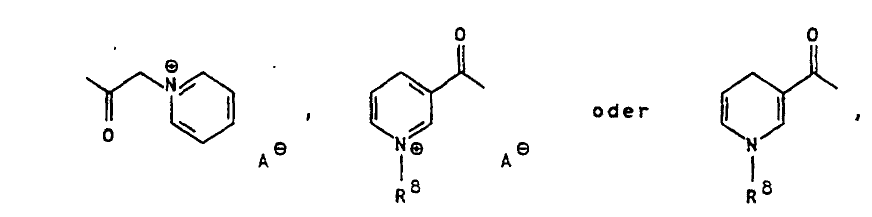 den Rest R⁹NH-CO-, den Rest R⁹NH-CS- oder Benzyloxycarbonyl steht;
- R^{4a}: für Wasserstoff, (C₁-C₄)-Alkylmercapto, den Rest -S(O)ₘR¹⁰, wobei m für die Zahlen 1 oder 2 steht, den Rest -NR¹¹R¹² oder den Rest -OR¹³ steht oder
- R³ und R^{4a}: zusammen für die Gruppierung -CO-O- stehen, wobei deren Carbonyl-C-Atom an die 5-Position des Pteridinmoleküls gebunden ist;
- R⁵: und R⁶ unabhängig voneinander für Wasserstoff oder Methyl stehen;
- R⁷: für Wasserstoff oder Methyl steht;
- R⁸: für (C₁-C₁₀)-Alkyl oder Benzyl steht;
- R⁹: für Wasserstoff, (C₁-C₆)-Alkyl, Cyclohexyl, Phenyl oder Benzoyl steht;
- R¹⁰: für Methyl steht;
- R¹¹ und R¹²: unabhängig voneinander für Wasserstoff oder Methyl stehen;
- R¹³: für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
- A^{θ}: für ein pharmakologisch verträgliches Anion steht;
und ihrer tautomeren Formen sowie ihrer pharmakologisch verträglichen Salze zur Herstellung eines Medikaments zur Vorbeugung und Behandlung von Krankheiten, die durch einen erhöhen Stickstoffmonoxid-Spiegel bedingt sind.

Besonders bevorzugt erfindungsgemäß zu verwendende Verbindungen der allgemeinen Formel I sind diejenigen, in denen R¹, R², R³, R⁵, R⁶ und R⁷ gleichzeitig für Wasserstoff stehen und R⁴ für den Rest R^{4a}-CH₂- steht und darin R^{4a} für eine Aminogruppe, eine Methylaminogruppe, eine Dimethylaminogruppe, eine (C₁-C₁₀)-Alkyloxygruppe oder eine Acetoxygruppe steht. Besonders bevorzugt zu verwenden sind weiterhin diejenigen, in denen R², R⁴, R⁵, R⁶ und R⁷ gleichzeitig für Wasserstoff stehen, R¹ für Wasserstoff oder Isobutyryl steht und R³ für einen Acylrest oder einen (Thio-)Carbamoylrest steht. Eine weitere Gruppe besonders bevorzugt zu verwendender Verbindungen der allgemeinen Formel I sind solche, in denen R², R⁶ und R⁷ gleichzeitig für Wasserstoff stehen, R¹ für Wasserstoff oder Isobutyryl steht, R³ für einen Acylrest steht, R⁴ für Phenyl oder durch eine Acetylaminogruppe, insbesondere eine para-ständige Acetylaminogruppe, substituiertes Phenyl steht und R⁵ für Wasserstoff oder Phenyl steht.

Die Verbindungen der allgemeinen Formel I sind bekannt und können nach oder analog zu bekannten Verfahren hergestellt werden. Bekannte Synthesemethoden für Pteridin-Derivate der allgemeinen Formel I sind z.B. die Methode von Gabriel-Isay oder die Taylor-Methode (siehe z.B. D.J.Brown, Fused Pyrimidines III, Pteridines (E.C.Taylor und A.Weissberger (Ed.), Wiley & Sons, New York)). Im einzelnen ist die Herstellung von Verbindungen der allgemeinen Formel I z.B. beschrieben in der EP-A-108 890, in der Dissertationsschrift von Hermann Michael Traub (Dissertation der Universität Konstanz, Deutschland (1987)), oder in J. Med. Chem. 30 (1987), 40.

Die erfindungsgemäß zu verwendenden Verbindungen der allgemeinen Formel I können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Verbindungen der allgemeinen Formel I können ein oder mehrere, z.B. zwei oder drei, insbesondere zwei, Säureäquivalente addieren. Die Säureadditionssalze können wie üblich durch Vereinigung der Komponenten, zweckmäßigerweise in einem geeigneten Lösungs- oder Verdünnungsmittel, hergestellt werden. Durch Anionenaustausch können Säureadditionssalze ineinander überführt werden. A^{θ} kann ebenso für ein Anion einer der genannten Säuren stehen. Verbindungen der allgemeinen Formel I, die saure Gruppen enthalten, können mit anorganischen oder organischen Basen Salze bilden. Beispielsweise für solche Salze sind z.B. Alkalimetallsalze, insbesondere Natrium- und Kaliumsalze, oder Ammoniumsalze, insbesondere solche mit organischen Resten am Ammoniumstickstoff.

Die Hemmung der NO-Freisetzung durch die Verbindungen der allgemeinen Formel I kann durch einen Aktivitätsassay bestimmt werden, der auf Arbeiten von Bredt und Snyder sowie Schmidt et al. basiert (s. D.S.Bredt und S.S. Snyder, Isolation of nitric oxide synthase, a calmodulin-requiring enzyme, Proc.Natl.Acad.Sci. USA 87 (1990), 682; H.H.H.W.Schmidt et al., Purification of a soluble isoform of guanylyl cyclase-activating factor synthase, Proc. Natl.Acad.Sci. USA 88 (1991), 365). Hierbei wird für gereinigte NO-Synthase (NOS) das bei der NO-Bildung anfallende Coprodukt L-Citrullin quantitativ erfaßt. Dies geschieht durch den Einsatz von ³H-radiomarkiertem L-Arginin als Substrat der Enzymreaktion, das zu ³H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillationsmessung ermittelte ³H-Aktivität entspricht dann der Menge an L-Citrullin. Einzelheiten der Durchführung sind weiter unten angegeben.

Krankheiten, die durch einen erhöhten NO-Spiegel entstehen und die somit erfindungsgemäß mit den Verbindungen der allgemeinen Formel I behandelt werden können bzw. denen mit diesen vorgebeugt werden kann, sind insbesondere pathologische Blutdruckabfälle, wie sie beim septischen oder hämorrhagischen Schock, bei der Tumor- bzw. Krebstherapie mit Cytokinen oder bei der Leberzirrhose auftreten. Des weiteren entzündliche Erkrankungen, wie rheumatoide Arthritis und insbesondere Colitis ulcerosa, sowie Insulin-abhängiger Diabetes mellitus und Transplantat-Abstoßungsreaktionen.

Aber auch die folgenden Erkrankungen stehen im Zusammenhang mit einer gesteigerten Produktion von Stickstoffmonoxid und können erfindungsgemäß behandelt bzw. vorgebeugt werden. Im Bereich Herz-Kreislauf sind dies Arteriosklerose, postischämische Gewebeschäden und Infarktschäden, Reperfusionsschäden, Myokarditis auf der Grundlage einer Coxsackie-Virus-Infektion und Kardiomyopathie; im Bereich Nervensystem/Zentrales Nervensystem Neuritiden unterschiedlicher Ätiogenese (Neuritisformen), Enzephalomyelitiden, virale neurodegenerative Erkrankungen, Morbus Alzheimer, Hyperalgesie, Epilepsie und Migräne; im Bereich Niere akutes Nierenversagen sowie Nephritiden unterschiedlicher Ätiogenese, speziell Glomerulonephritis.

Außerdem sind auch Behandlungen im Bereich des Magens und des Uterus/der Plazenta sowie eine Beeinflussung der Motilität der Spermien Anwendungsgebiete für die Verbindungen der allgemeinen Formel I.

Die Verbindungen der allgemeinen Formel I und ihre pharmakologisch annehmbaren Salze können als Hilfsstoffe in biochemischen und pharmakologischen Untersuchungen in der Forschung und in Diagnoseverfahren eingesetzt werden, und sie können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen, enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen oder Infusionslösungen, oder perkutan, z.B. in Form von Salben oder Tinkturen, erfolgen.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon; entzündungshemmende Substanzen, wie etwa Corticosteroide, Salicylate oder Propionsäurederivate, wie beispielsweise Ibuprofen; Antibiotika, wie z.B. Penicilline oder Cephalosporine; NO-Donoren, wie z.B. organische Nitrate oder Sydnonimine oder Furoxane.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, in ähnlichen Mengenbereichen, d.h. im allgemeinen ebenfalls bei 0,5 bis 100 mg/Mensch. Die Tagesdosis kann in mehrere, z.B. 2 bis 4, Teilverabreichungen aufgeteilt werden.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Für verschiedene Pteridin-Derivate der allgemeinen Formel I sind bisher keine pharmakologischen Wirkungen oder medizinischen Verwendungen bekannt gewesen. Für solche Verbindungen gibt die vorliegende Erfindung die erste medizinische Indikation an. Gegenstand der vorliegenden Erfindung sind auch Pteridin-Derivaten der allgemeinen Formel I, 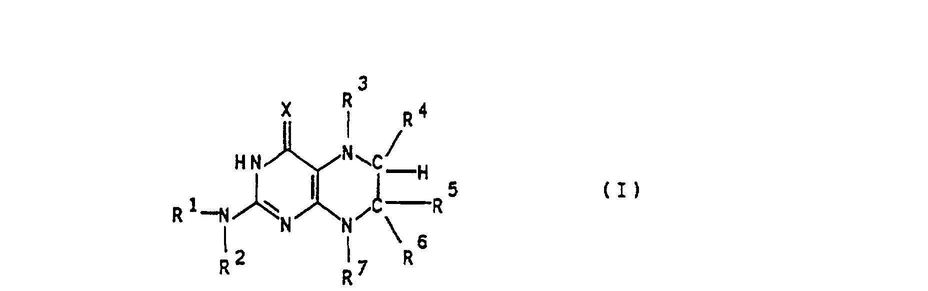 in der
- X: für O oder NH steht;
- R¹: für Wasserstoff, Methyl, (C₁-C₅)-Alkanoyl, Nicotinoyl oder (1-Methyl-3-pyridinio)carbonyl steht;
- R²: für Wasserstoff oder Methyl steht;
- R³: für Wasserstoff, Methyl, Ethyl, Benzyl, (C₁-C₅)-Alkanoyl, unsubstituiertes Benzoyl, substituiertes Benzoyl, Pyridoyl, Thienylcarbonyl, einen der Reste 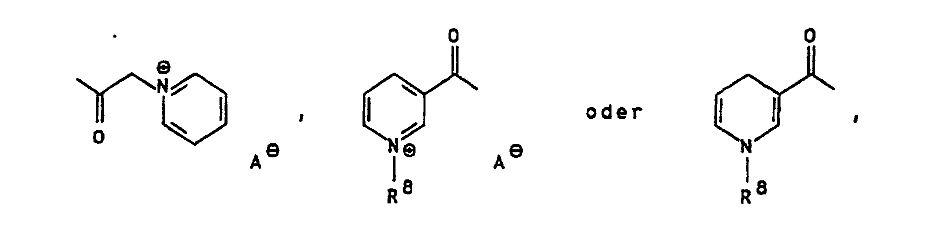 den Rest R⁹R^{9a}N-CO-, den Rest R⁹R^{9a}N-CS-, Phenoxycarbonyl oder Benzyloxycarbonyl steht;
- R⁴: für Wasserstoff, (C₂-C₅)-Alkyl, unsubstituiertes Phenyl, substituiertes Phenyl oder den Rest R^{4a}-CH₂-steht:
- R^{4a}: für Wasserstoff, (C₁-C₄)-Alkylmercapto, den Rest -S(O)ₘR¹⁰, wobei m für die Zahlen 1 oder 2 steht, den. Rest -NR¹¹R¹² oder den Rest -OR¹³ steht oder
- R³ und R^{4a}: zusammen für die Gruppierung -CO-O- stehen, wobei deren Carbonyl-C-Atom an die 5-Position des Pteridinmoleküls gebunden ist;
- R⁵: für Wasserstoff, Methyl oder Phenyl steht;
- R⁶: für Wasserstoff oder Methyl steht;
- R⁷: für Wasserstoff oder Methyl steht;
- R⁸: für (C₁-C₁₀)-Alkyl oder Benzyl steht;
- R⁹: für Wasserstoff, (C₁-C₆)-Alkyl, Cyclohexyl, Phenyl oder Benzoyl steht;
- R^{9a}: für Wasserstoff, Methyl oder Ethyl steht;
- R¹⁰: für Methyl steht;
- R¹¹ und R¹²: unabhängig voneinander für Wasserstoff oder Methyl stehen;
- R¹³: für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
- A^{θ}: für ein pharmakologisch verträgliches Anion steht;
und ihrer tautomeren Formen sowie ihrer pharmakologisch verträglichen Salze, wobei aber, wenn X für Sauerstoff und gleichzeitig R⁴ für den Rest R^{4a}-CH₂- steht, mindestens einer der Substituenten R¹, R², R³ und R⁷ eine andere Bedeutung als Wasserstoff hat, als pharmakolosche Wirkstoffe.

Die folgenden Beispiele geben Verbindungen der allgemeinen Formel I an, die erfindungsgemäß eingesetzt werden können. In der Rubrik "Salz" ist angegeben, wieviel Mol Säure und wieviel Mol Kristallwasser gegebenenfalls pro Mol Wirkstoff in den Pteridin-Derivaten der allgemeinen Formel I vorlagen. In den Beispielen werden folgende Abkürzungen verwendet:
Me = Methyl
Et = Ethyl
iPr = Isopropyl
iBu = Isobutyl
tBu = tert-Butyl
Ph = Phenyl
Py = 3-Pyridyl

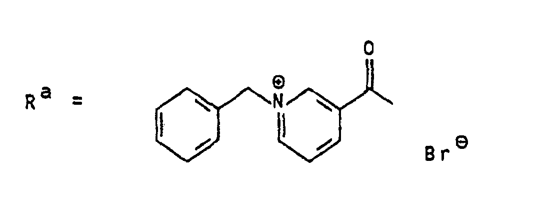

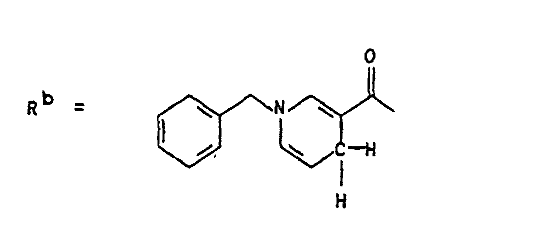

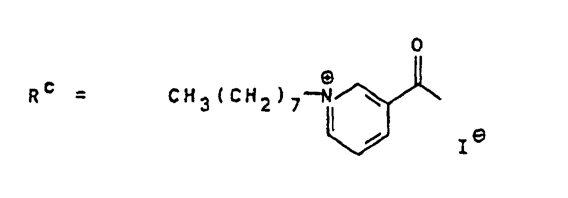

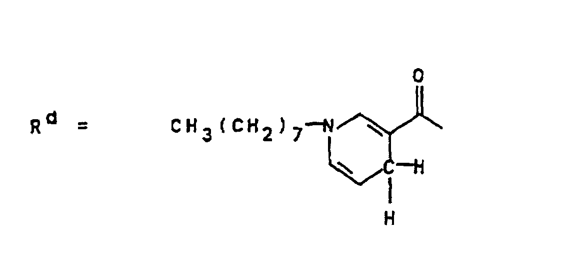

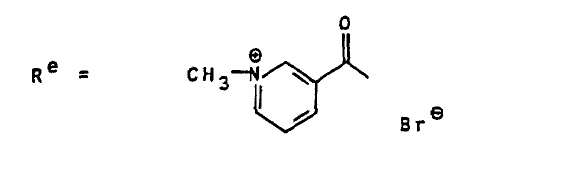

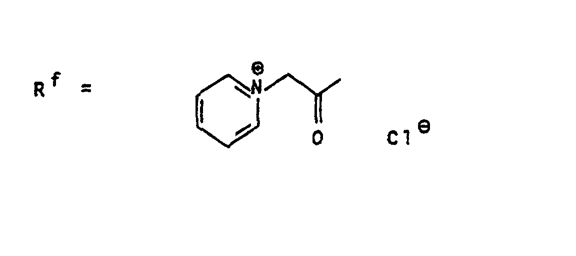

### Beispiel 1

| | | | | | |
|---|---|---|---|---|---|
| X = O | R¹ = H | R² = H | R⁵ = Me | R⁶ = H | R⁷ = H |
| R³ = H | | R⁴ = Me | | | |

### Beispiel 2

| | | | | | |
|---|---|---|---|---|---|
| X = O | R¹ = H | R² = H | R⁵ = H | R⁶ = H | R⁷ = Me |
| R³ = H | | R⁴ = Me | | | |

### Beispiel 3

| | | | | | |
|---|---|---|---|---|---|
| X = O | R¹ = H | R² = H | R⁵ = H | R⁶ = H | R⁷ = Me |
| R³ = CHO | | R⁴ = Me | | | |

### Beispiel 4

| | | | | | |
|---|---|---|---|---|---|
| X = O | R¹ = H | R² = H | R⁵ = Me | R⁶ = H | R⁷ = Me |
| R³ = CHO | | R⁴ = Me | | | |

### Beispiel 5

| | | | | | |
|---|---|---|---|---|---|
| X = O | R¹ = H | R² = H | R⁵ = Me | R⁶ = H | R⁷ = H |
| R³ = CONH₂ | | R⁴ = Me | | | |
| Salz: 0,5H₂O | | Schmp. 257°C | | | |

### Beispiel 6

| | | | | | |
|---|---|---|---|---|---|
| X = O | R¹ = H | R² = H | R⁵ = Me | R⁶ = H | R⁷ = H |
| R³ = CSNHMe | | R⁴ = Me | | | |
| Salz: 1,5H₂O | | Schmp. 195°C | | | |

### Beispiel 7

| | | | | | |
|---|---|---|---|---|---|
| X = O | R¹ = H | R² = H | R⁵ = Me | R⁶ = H | R⁷ = H |
| R³ = CSNHPh | | R⁴ = Me | | | |
| Salz: 2H₂O | Schmp. 239°C | | | | |

### Beispiel 8

| | | | | | |
|---|---|---|---|---|---|
| X = O | R¹ = H | R² = H | R⁵ = Me | R⁶ = H | R⁷ = H |
| R³ = CSNHEt | | R⁴ = Me | | | |
| Salz: 2H₂O | | Schmp.202°C | | | |

### Beispiel 9

| | | | | | |
|---|---|---|---|---|---|
| X = O | R¹ = H | R² = H | R⁵ = Me | R⁶ = H | R⁷ = H |
| R³ = CSNHCOPh | | R⁴ = Me | | | |
| Salz: 0,5 H₂O | | Schmp. 229°C | | | |

### Beispiel 10

| | | | | | |
|---|---|---|---|---|---|
| X = O | R¹ = H | R² = H | R⁵ = Me | R⁶ = H | R⁷ = H |
| R³ = CONHPh | | R⁴ = Me | | | |
| Salz: 1H₂O | | Schmp. 197°C (Zers.) | | | |

### Beispiel 11

| | | | | | |
|---|---|---|---|---|---|
| X = O | R¹ = H | R² = H | R⁵ = Me | R⁶ = H | R⁷ = H |
| R³ = CONHtBu | | R⁴ = Me | | | |
| Salz: 1H₂O | | Schmp. 260°C (Zers.) | | | |

### Beispiel 12

| | | | | | |
|---|---|---|---|---|---|
| X = O | R¹ = H | R² = H | R⁵ = Me | R⁶ = H | R⁷ = H |
| R³ = CONH-Cyclohexyl | | | R⁴ = Me | | |
| Salz: 1H₂O | | Schmp. 242°C | | | |

### Beispiel 13

| | | | | | |
|---|---|---|---|---|---|
| X = 0 | R¹ = H | R² = H | R⁵ = Me | R⁶ = H | R⁷ = H |
| R³ = COPh | | R⁴ = Me | | | |
| | | Schmp. >320°C | | | |

### Beispiel 14

| | | | | | |
|---|---|---|---|---|---|
| X = O | R¹ = H | R² = H | R⁵ = Me | R⁶ = H | R⁷ = H |
| R³ = COPy | | R⁴ = Me | | | |
| | | Schmp. 295°C | | | |

### Beispiel 15

| | | | | | |
|---|---|---|---|---|---|
| X = O | R¹ = H | R² = H | R⁵ = Me | R⁶ = H | R⁷ = H |
| R³ = R^{a} | | R⁴ = Me | | | |
| Salz: 0,5H₂O | | Schmp. 275°C | | | |

### Beispiel 16

| | | | | | |
|---|---|---|---|---|---|
| X = O | R¹ = H | R² = H | R⁵ = Me | R⁶ = H | R⁷ = H |
| R³ = R^{b} | | R⁴ = Me | | | |
| | | Schmp. 255°C | | | |

### Beispiel 17

| | | | | | |
|---|---|---|---|---|---|
| X = O | R¹ = H | R² = H | R⁵ = H | R⁶ = H | R⁷ = H |
| R³ = R^{b} | | R⁴ = Me | | | |
| Salz: 1H₂O | | Schmp. 232°C | | | |

### Beispiel 18

| | | | | | |
|---|---|---|---|---|---|
| X = O | R¹ = H | R² = H | R⁵ = Me | R⁶ = H | R⁷ = H |
| R³ = R^{c} | | R⁴ = Me | | | |
| Salz: 0,5H₂O | | Schmp. 180°C | | | |

### Beispiel 19

| | | | | | |
|---|---|---|---|---|---|
| X = O | R¹ = H | R² = H | R⁵ = H | R⁶ = H | R⁷ = H |
| R³ = COPy | | R⁴ = Me | | | |
| Salz: 1H₂O | | Schmp. 265-270°C | | | |

### Beispiel 20

| | | | | | |
|---|---|---|---|---|---|
| X = O | R¹ = H | R² = H | R⁵ = H | R⁶ = H | R⁷ = H |
| R³ = R^{c} | | R⁴ = Me | | | |
| | | Schmp. 206-208°C | | | |

### Beispiel 21

| | | | | | |
|---|---|---|---|---|---|
| X = O | R¹ = H | R² = H | R⁵ = Me | R⁶ = H | R⁷ = H |
| R³ = Rd | | R⁴ = Me | | | |
| | | Schmp. >200°C (Zers.) | | | |

### Beispiel 22

| | | | | |
|---|---|---|---|---|
| X = 0 | R¹ = COiPr | R² = H | R⁵ = Me | R⁶ = H |
| R⁷ = H | R³ = COPy | R⁴ = Me | | |
| | | Schmp. >300°C | | |

### Beispiel 23

| | | | | |
|---|---|---|---|---|
| X = 0 | R¹ = COPy | R² = H | R⁵ = Me | R⁶ = H |
| R⁷ = H | R³ = COPy | R⁴ = Me | | |
| | | Schmp. 325°C | | |

### Beispiel 24

| | | | | | |
|---|---|---|---|---|---|
| X = 0 | R¹ = Me | R² = Me | R⁵ = Me | R⁶ = H | R⁷ = H |
| R³ = H | | R⁴ = Me | | | |
| Salz: 2HCl | | Schmp. >300°C | | | |

### Beispiel 25

| | | | | | |
|---|---|---|---|---|---|
| X = O | R¹ = Me | R² = Me | R⁵ = Me | R⁶ = H | R⁷ = H |
| R³ = COPy | | R⁴ = Me | | | |
| | | Schmp. 315°C (Zers.) | | | |

### Beispiel 26

| | | | | | |
|---|---|---|---|---|---|
| X = O | R¹ = Me | R² = Me | R⁵ = Me | R⁶ = H | R⁷ = H |
| R³ = Re | | R⁴ = Me | | | |
| | | Schmp. 238-240°C | | | |

### Beispiel 27

| | | | | | |
|---|---|---|---|---|---|
| X = O | R¹ = Me | R² = Me | R⁵ = Me | R⁶ = H | R⁷ = H |
| R³ = R^{a} | | R⁴ = Me | | | |
| Salz: 0,5H₂O | | Schmp. 271°C | | | |

### Beispiel 28

| | | | | |
|---|---|---|---|---|
| X = O | R¹ = R^{e} | R² = H | R⁵ = Me | R⁶ = H |
| R⁷ = H | R³ = R^{e} | R⁴ = Me | | |
| | | Schmp. 250°C (Zers.) | | |

### Beispiel 29

| | | | | |
|---|---|---|---|---|
| X = O | R¹ = COiPr | R² = H | R⁵ = Me | R⁶ = H |
| R⁷ = H | R³ = R^{a} | R⁴ = Me | | |
| Salz: 0,5H₂O | Schmp. 251°C | | | |

### Beispiel 30

| | | | | | |
|---|---|---|---|---|---|
| X = O | R¹ = H | R² = H | R⁵ = H | R⁶ = H | R⁷ = H |
| R³ = H | | R⁴ = CH₂OH | | | |
| Salz: 2HCl+0,5H₂O | | | Schmp. 238-240°C (Zers.) | | |

### Beispiel 31

| | | | | | |
|---|---|---|---|---|---|
| X = O | R¹ = H | R² = H | R⁵ = H | R⁶ = H | R⁷ = H |
| R³ = COPh | | R⁴ = CH₂OH | | | |
| | | Schmp. 246°C (Zers.) | | | |

### Beispiel 32

| | | | | | |
|---|---|---|---|---|---|
| X = 0 | R¹ = H | R² = H | R⁵ = H | R⁶ = H | R⁷ = Me |
| R³ = H | | R⁴ = CH₂OH | | | |

### Beispiel 33

| | | | | | |
|---|---|---|---|---|---|
| X = O | R¹ = H | R² = H | R⁵ = H | R⁶ = H | R⁷ = Me |
| R³ = CHO | | R⁴ = CH₂OH | | | |

### Beispiel 34

| | | | | | |
|---|---|---|---|---|---|
| X = O | R¹ = H | R² = H | R⁵ = H | R⁶ = H | R⁷ = Me |
| R³ = COMe | | R⁴ = CH₂OH | | | |

### Beispiel 35

| | | | | | |
|---|---|---|---|---|---|
| X = O | R¹ = H | R² = H | R⁵ = H | R⁶ = H | R⁷ = H |
| R³ = H | | R⁴ = CH₂OMe | | | |
| Salz: 2HCl | | Schmp. 170°C (Zers.) | | | |

### Beispiel 36

| | | | | | |
|---|---|---|---|---|---|
| X = O | R¹ = H | R² = H | R⁵ = H | R⁶ = H | R⁷ = H |
| R³ = H | | R⁴ = CH₂OEt | | | |
| Salz: 2HCl | | Schmp. 165°C (Zers.) | | | |

### Beispiel 37

| | | | | | |
|---|---|---|---|---|---|
| X = O | R¹ = H | R² = H | R⁵ = H | R⁶ = H | R⁷ = H |
| R³ = H | | R⁴ = CH₂OiPr | | | |
| Salz: 2HCl | | Schmp. 219-221°C (Zers.) | | | |

### Beispiel 38

| | | | | | |
|---|---|---|---|---|---|
| X = O | R¹ = H | R² = H | R⁵ = H | R⁶ = H | R⁷ = H |
| R³ = H | | R⁴ = CH₂OiBu | | | |
| Salz: 2HCl+0,5H₂O | | | Schmp. 232-234°C (Zers.) | | |

### Beispiel 39

| | | | | | |
|---|---|---|---|---|---|
| X = O | R¹ = H | R² = H | R⁵ = H | R⁶ = H | R⁷ = H |
| R³ = H | | R⁴ = CH₂O-n-Butyl | | | |

### Beispiel 40

| | | | | | |
|---|---|---|---|---|---|
| X = O | R¹ = H | R² = H | R⁵ = H | R⁶ = H | R⁷ = H |
| R³ = H | | R⁴ = CH₂OtBu | | | |

### Beispiel 41

| | | | | | |
|---|---|---|---|---|---|
| X = O | R¹ = H | R² = H | R⁵ = H | R⁶ = H | R⁷ = H |
| R³ = H | | R⁴ = CH₂O-n-Pentyl | | | |

### Beispiel 42

| | | | | | |
|---|---|---|---|---|---|
| X = O | R¹ = H | R² = H | R⁵ = H | R⁶ = H | R⁷ = H |
| R³ = H | | R⁴ = CH₂O-n-Octyl | | | |

### Beispiel 43

| | | | | | |
|---|---|---|---|---|---|
| X = O | R¹ = H | R² = H | R⁵ = H | R⁶ = H | R⁷ = H |
| R³ = H | | R⁴ = CH₂O-n-Decyl | | | |
| Salz: 2HCl | | Schmp. 180°C | | | |

### Beispiel 44

| | | | | | |
|---|---|---|---|---|---|
| X = O | R¹ = H | R² = H | R⁵ = H | R⁶ = H | R⁷ = H |
| R³ = H | | R⁴ = CH₂OCH₂CH₂OMe | | | |
| Salz: 2HCl | | Schmp. 221-222°C | | | |

### Beispiel 45

| | | | | | |
|---|---|---|---|---|---|
| X = O | R¹ = H | R² = H | R⁵ = H | R⁶ = H | R⁷ = H |
| R³ = H | | R⁴ = CH₂OPh | | | |
| Salz: 2HCl+0,25H₂O | | Schmp. 254-256°C (Zers.) | | | |

### Beispiel 46

| | | | | | |
|---|---|---|---|---|---|
| X = O | R¹ = H | R² = H | R⁵ = Me | R⁶ = Me | R⁷ = H |
| R³ = H | | R⁴ = CH₂OH | | | |

### Beispiel 47

| | | | | | |
|---|---|---|---|---|---|
| X = O | R¹ = H | R² = H | R⁵ = Me | R⁶ = Me | R⁷ = H |
| R³ = H | | R⁴ = CH₂OMe | | | |

### Beispiel 48

| | | | | | |
|---|---|---|---|---|---|
| X = O | R¹ = H | R² = H | R⁵ = Me | R⁶ = Me | R⁷ = H |
| R³ = H | | R⁴ = CH₂OEt | | | |

### Beispiel 49

| | | | | | |
|---|---|---|---|---|---|
| X = 0 | R¹ = H | R² = H | R⁵ = Me | R⁶ = Me | R⁷ = H |
| R³ = H | | R⁴ = CH₂O-n-Propyl | | | |

### Beispiel 50

| | | | | | |
|---|---|---|---|---|---|
| X = O | R¹ = H | R² = H | R⁵ = Me | R⁶ = Me | R⁷ = H |
| R³ = H | | R⁴ = CH₂OiPr | | | |

### Beispiel 51

| | | | | | |
|---|---|---|---|---|---|
| X = O | R¹ = H | R² = H | R⁵ = Me | R⁶ = Me | R⁷ = H |
| R³ = H | | R⁴ = CH₂O-n-Pentyl | | | |

### Beispiel 52

| | | | | | |
|---|---|---|---|---|---|
| X = O | R¹ = H | R² = H | R⁵ = Me | R⁶ = Me | R⁷ = H |
| R³ = H | | R⁴ = CH₂O-n-Octyl | | | |

### Beispiel 53

| | | | | | |
|---|---|---|---|---|---|
| X = O | R¹ = H | R² = H | R⁵ = Me | R⁶ = Me | R⁷ = H |
| R³ = H | | R⁴ = CH₂O-CH₂CH₂CH(CH₃)₂ | | | |

### Beispiel 54

| | | | | | |
|---|---|---|---|---|---|
| X = O | R¹ = H | R² = H | R⁵ = Me | R⁶ = Me | R⁷ = H |
| R³ = H | | R⁴ = CH₂O-(CH₂)₃Ph | | | |

### Beispiel 55

| | | | | | |
|---|---|---|---|---|---|
| X = O | R¹ = H | R² = H | R⁵ = Me | R⁶ = Me | R⁷ = H |
| R³ = H | | R⁴ = CH₂O-(CH₂)₃-Cyclohexyl | | | |

### Beispiel 56

| | | | | | |
|---|---|---|---|---|---|
| X = O | R¹ = H | R² = H | R⁵ = H | R⁶ = H | R⁷ = Me |
| R³ = CHO | | R⁴ = CH₂OCHO | | | |

### Beispiel 57

| | | | | | |
|---|---|---|---|---|---|
| X = O | R¹ = H | R² = H | R⁵ = H | R⁶ = H | R⁷ = H |
| R³ = H | | R⁴ = CH₂OCOMe | | | |
| Salz: H₂SO₄+0,25H₂O | | | Schmp. 230-232°C | | |

### Beispiel 58

| | | | | | |
|---|---|---|---|---|---|
| X = O | R¹ = H | R² = H | R⁵ = H | R⁶ = H | R⁷ = Me |
| R³ = COMe | | R⁴ = CH₂OCOMe | | | |

### Beispiel 59

| | | | | |
|---|---|---|---|---|
| X = O | R¹ = COMe | R² = H | R⁵ = H | R⁶ = H |
| R⁷ = H | R³ = CH₂Ph | R⁴ = CH₂OCOMe | | |

### Beispiel 60

| | | | | | |
|---|---|---|---|---|---|
| X = O | R¹ = H | R² = H | R⁵ = H | R⁶ = H | R⁷ = H |
| R³ = H | | R⁴ = CH₂OCOCH₂OH | | | |
| Salz: H₂SO₄ | | Schmp. 220°C (Zers.) | | | |

### Beispiel 61

| | | | | | |
|---|---|---|---|---|---|
| X = O | R¹ = H | R² = H | R⁵ = H | R⁶ = H | R⁷ = H |
| R³ = COtBu | | R⁴ = CH₂OCOtBu | | | |

### Beispiel 62

| | | | | |
|---|---|---|---|---|
| X = O | R¹ = COtBu | R² = H | R⁵ = H | R⁶ = H |
| R⁷ = H | R³ = COOCH₂Ph | R⁴ = CH₂OCOtBu | | |

### Beispiel 63

| | | | | | |
|---|---|---|---|---|---|
| X = O | R¹ = H | R² = H | R⁵ = H | R⁶ = H | R⁷ = H |
| R³ = H | | R⁴ = CH₂OCOOEt | | | |
| Salz: H₂SO₄ | | Schmp. 214°C | | | |

### Beispiel 64

| | | | | | |
|---|---|---|---|---|---|
| X = 0 | R¹ = H | R² = H | R⁵ = H | R° = H | R⁷ = H |
| R³ = H | | R⁴ = CH₂OCOCH(Me)(NH₂) (L) | | | |
| Salz: 1,5H₂SO₄+0,25H₂O | | | Schmp. 214°C (Zers.) | | |

### Beispiel 65

| | | | | | |
|---|---|---|---|---|---|
| X = O | R¹ = H | R² = H | R⁵ = H | R⁶ = H | R⁷ = H |
| R³ = H | | R⁴ = CH₂OCOCH(iPr)(NH₂) (L) | | | |
| Salz: 1,5H₂SO₄+H₂O | | | Schmp. 190°C (Zers.) | | |

### Beispiel 66

| | | | | | |
|---|---|---|---|---|---|
| X = 0 | R¹ = H | R² = H | R⁵ = H | R⁶ = H | R⁷ = H |
| R³ = H | | R⁴ = CH₂OCOCH(CH₂Ph)(NH₂) (DL) | | | |
| Salz: 2HCl+0,5H₂O | | | Schmp. 205°C (Zers.) | | |

### Beispiel 67

| | | | | | |
|---|---|---|---|---|---|
| X = O | R¹ = H | R² = H | R⁵ = H | R⁶ = H | R⁷ = H |
| R³ = Et | | R⁴ = CH₂SMe | | | |
| Salz: 2HCl+2/3H₂O | | | Schmp. 195-196°C (Zers.) | | |

### Beispiel 68

| | | | | | |
|---|---|---|---|---|---|
| X = O | R¹ = H | R² = H | R⁵ = H | R⁶ = H | R⁷ = H |
| R³ = H | | R⁴ = CH₂S(O)₂Me | | | |
| Salz: 2HCl | | Schmp. 252-253°C | | | |

### Beispiel 69

| | | | | | |
|---|---|---|---|---|---|
| X = O | R¹ = H | R² = H | R⁵ = H | R⁶ = H | R⁷ = H |
| R³ = H | | R⁴ = CH₂NH₂ | | | |
| Salz: 3HCl | | Schmp. 264-265°C (Zers.) | | | |

### Beispiel 70

| | | | | | |
|---|---|---|---|---|---|
| X = O | R¹ = H | R² = H | R⁵ = H | R⁶ = H | R⁷ = H |
| R³ = H | | R⁴ = CH₂NHMe | | | |
| Salz: 3HCl | | Schmp. 273-274°C (Zers.) | | | |

### Beispiel 71

| | | | | | |
|---|---|---|---|---|---|
| X = O | R¹ = H | R² = H | R⁵ = H | R⁶ = H | R⁷ = H |
| R³ = H | | R⁴ = CH₂NMe₂ | | | |
| Salz: 3HCl+0,5H₂O | | | Schmp. 224-225°C | | |

### Beispiel 72

| | | | | | |
|---|---|---|---|---|---|
| X = NH | R¹ = H | R² = H | R⁵ = H | R⁶ = H | R⁷ = H |
| R³ = H | | R⁴ = CH₂OH | | | |

### Beispiel 73

| | | | | |
|---|---|---|---|---|
| X = NH R¹ = H | R² = H | R⁵ = H | R⁶ = H | R⁷ = H |
| R³ = H | R⁴ = CH₂OEt | | | |
| Salz: 3HCl | Schmp. 130°C (Zers.) | | | |

### Beispiel 74

Verbindung der Formel

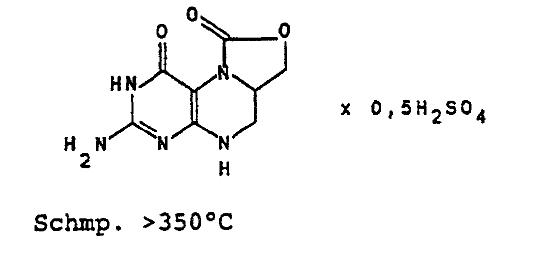

### Beispiel 75

| | | | | | |
|---|---|---|---|---|---|
| X = O | R¹ = H | R² = H | R⁵ = H | R⁶ = H | R⁷ = H |
| R³ = H | | R⁴ = CH₂SEt | | | |

### Beispiel 76

| | | | | | |
|---|---|---|---|---|---|
| X = O | R¹ = H | R² = H | R⁵ = H | R⁶ = H | R⁷ = H |
| R³ = H | | R⁴ = CH₂S-n-Propyl | | | |

### Beispiel 77

| | | | | | |
|---|---|---|---|---|---|
| X = O | R¹ = H | R² = H | R⁵ = H | R⁶ = H | R⁷ = H |
| R³ = H | | R⁴ = CH₂S-n-Butyl | | | |

### Beispiel 78

| | | | | | |
|---|---|---|---|---|---|
| X = O | R¹ = H | R² = H | R⁵ = H | R⁶ = H | R⁷ = Me |
| R³ = H | | R⁴ = H | | | |

### Beispiel 79

| | | | | | |
|---|---|---|---|---|---|
| X = O | R¹ = H | R² = H | R⁵ = H | R⁶ = H | R⁷ = Me |
| R³ = CHO | | R⁴ = H | | | |

### Beispiel 80

| | | | | | |
|---|---|---|---|---|---|
| X = O | R¹ = H | R² = H | R⁵ = H | R⁶ = H | R⁷ = H |
| R³ = CSNHPh | | R⁴ = H | | | |
| | | Schmp. >300°C | | | |

### Beispiel 81

| | | | | | |
|---|---|---|---|---|---|
| X = O | R¹ = H | R² = H | R⁵ = H | R⁶ = H | R⁷ = H |
| R³ = CSNHCOPh | | R⁴ = H | | | |
| | | Schmp. >220°C (Zers.) | | | |

### Beispiel 82

| | | | | | |
|---|---|---|---|---|---|
| X = O | R¹ = H | R² = H | R⁵ = H | R⁶ = H | R⁷ = H |
| R³ = COPh | | R⁴ = H | | | |
| | | Schmp. >300°C | | | |

### Beispiel 83

| | | | | | |
|---|---|---|---|---|---|
| X = O | R¹ = H | R² = H | R⁵ = H | R⁶ = H | R⁷ = H |
| R³ = COPy | | R⁴ = CH₂CH₂CH₃ | | | |
| | | Schmp. 298°C | | | |

### Beispiel 84

| | | | | |
|---|---|---|---|---|
| X = NH R¹ = H | R² = H | R⁵ = H | R⁶ = H | R⁷ = H |
| R³ = R^{f} | R⁴ = H | | | |
| | Schmp. >300°C | | | |

### Beispiel 85

| | | | | | |
|---|---|---|---|---|---|
| X = O | R¹ = COtBu | R² = H | R⁵ = Ph | R⁶ = H | R⁷ = H |
| R³ = R^{b} | | R⁴ = Ph | | | |
| | | Schmp. 240°C (Zers.) | | | |

### Beispiel 86

| | | | | | |
|---|---|---|---|---|---|
| X = O | R¹ = COiPr | R² = H | R⁵ = H | R⁶ = H | R⁷ = H |
| R³ = CO-(2-Chlorphenyl) | | | R⁴ = H | | |

### Beispiel 87

| | | | | | |
|---|---|---|---|---|---|
| X = O | R¹ = COiPr | R² = H | R⁵ = H | R⁶ = H | R⁷ = H |
| R³ = CO-(3-Chlorphenyl) | | | R⁴ = H | | |

### Beispiel 88

| | | | | | |
|---|---|---|---|---|---|
| X = O | R¹ = COiPr | R² = H | R⁵ = H | R⁶ = H | R⁷ = H |
| R³ = CO-(4-Chlorphenyl) | | | R⁴ = H | | |

### Beispiel 89

| | | | | | |
|---|---|---|---|---|---|
| X = O | R¹ = COiPr | R² = H | R⁵ = H | R⁶ = H | R⁷ = H |
| R³ = CO-(2-Fluorphenyl) | | | R⁴ = H | | |

### Beispiel 90

| | | | | | |
|---|---|---|---|---|---|
| X = O | R¹ = COiPr | R² = H | R⁵ = H | R⁶ = H | R⁷ = H |
| R³ = CO-(3-Fluorphenyl) | | | R⁴ = H | | |

### Beispiel 91

| | | | | | |
|---|---|---|---|---|---|
| X = 0 | R¹ = COiPr | R² = H | R⁵ = H | R⁶ = H | R⁷ = H |
| R³ = CO-(4-Fluorphenyl) | | | R⁴ = H | | |

### Beispiel 92

| | | | | | |
|---|---|---|---|---|---|
| X = 0 | R¹ = COiPr | R² = H | R⁵ = H | R⁶ = H | R⁷ = H |
| R³ = CO-(4-Iodphenyl) | | | R⁴ = H | | |

### Beispiel 93

| | | | | | |
|---|---|---|---|---|---|
| X = O | R¹ = COiPr | R² = H | R⁵ = H | R⁶ = H | R⁷ = H |
| R³ = CO-(2,3,4-Trimethoxyphenyl) | | | | R⁴ = H | |

### Beispiel 94

| | | | | | |
|---|---|---|---|---|---|
| X = O | R¹ = COiPr | R² = H | R⁵ = H | R⁶ = H | R⁷ = H |
| R³ = CO-(2-Methoxyphenyl) | | | R⁴ = H | | |

### Beispiel 95

| | | | | | |
|---|---|---|---|---|---|
| X = 0 | R¹ = COiPr | R² = H | R⁵ = H | R⁶ = H | R⁷ = H |
| R³ = COOPh | | R⁴ = H | | | |

### Beispiel 96

| | | | | | |
|---|---|---|---|---|---|
| X = 0 | R¹ = COiPr | R² = H | R⁵ = H | R⁶ = H | R⁷ = H |
| R³ = CONMe₂ | | R⁴ = H | | | |

### Beispiel 97

| | | | | | |
|---|---|---|---|---|---|
| X = O | R¹ = COiPr | R² = H | R⁵ = H | R⁶ = H | R⁷ = H |
| R³ = CO-(2-Thienyl) | | R⁴ = H | | | |

### Beispiel 98

| | | | | | |
|---|---|---|---|---|---|
| X = 0 | R¹ = H | R² = H | R⁵ = H | R⁶ = H | R⁷ = H |
| R³ = CO-(2-Chlorphenyl) | | | R⁴ = H | | |

### Beispiel 99

| | | | | | |
|---|---|---|---|---|---|
| X = O | R¹ = H | R² = H | R⁵ = H | R⁶ = H | R⁷ = H |
| R³ = CO-(3-Chlorphenyl) | | | R⁴ = H | | |

### Beispiel 100

| | | | | | |
|---|---|---|---|---|---|
| X = O | R¹ = H | R² = H | R⁵ = H | R⁶ = H | R⁷ = H |
| R³ = CO-(4-Chlorphenyl) | | | R⁴ = H | | |

### Beispiel 101

| | | | | | |
|---|---|---|---|---|---|
| X = O | R¹ = H | R² = H | R⁵ = H | R⁶ = H | R⁷ = H |
| R³ = CO-(2-Fluorphenyl) | | | R⁴ = H | | |

### Beispiel 102

| | | | | | |
|---|---|---|---|---|---|
| X = O | R¹ = H | R² = H | R⁵ = H | R⁶ = H | R⁷ = H |
| R³ = CO-(3-Fluorphenyl) | | | R⁴ = H | | |

### Beispiel 103

| | | | | | |
|---|---|---|---|---|---|
| X = O | R¹ = H | R² = H | R⁵ = H | R⁶ = H | R⁷ = H |
| R³ = CO-(4-Fluorphenyl) | | | R⁴ = H | | |

### Beispiel 104

| | | | | | |
|---|---|---|---|---|---|
| X = O | R¹ = H | R² = H | R⁵ = H | R⁶ = H | R⁷ = H |
| R³ = CO-(4-Iodphenyl) | | | R⁴ = H | | |

### Beispiel 105

| | | | | | |
|---|---|---|---|---|---|
| X = O | R¹ = H | R² = H | R⁵ = H | R⁶ = H | R⁷ = H |
| R³ = CO-(2,3,4-Trimethoxyphenyl) | | | | R⁴ = H | |

### Beispiel 106

| | | | | | |
|---|---|---|---|---|---|
| X = O | R¹ = H | R² = H | R⁵ = H | R⁶ = H | R⁷ = H |
| R³ = CO-(2-Methoxyphenyl) | | | R⁴ = H | | |

### Beispiel 107

| | | | | | |
|---|---|---|---|---|---|
| X = O | R¹ = H | R² = H | R⁵ = H | R⁶ = H | R⁷ = H |
| R³ = COOPh | | R⁴ = H | | | |

### Beispiel 108

| | | | | | |
|---|---|---|---|---|---|
| X = O | R¹ = H | R² = H | R⁵ = H | R⁶ = H | R⁷ = H |
| R³ = CONMe₂ | | R⁴ = H | | | |

### Beispiel 109

| | | | | | |
|---|---|---|---|---|---|
| X = O | R¹ = H | R² = H | R⁵ = H | R⁶ = H | R⁷ = H |
| R³ = CO-(2-Thienyl) | | | R⁴ = H | | |

### Beispiel 110

| | | | | | |
|---|---|---|---|---|---|
| X = O | R¹ = H | R² = H | R⁵ = H | R⁶ = H | R⁷ = H |
| R³ = CO-(4-tert-Butylphenyl) | | | R⁴ = H | | |

### Beispiel 111

| | | | | | |
|---|---|---|---|---|---|
| X = O | R¹ = H | R² = H | R⁵ = H | R⁶ = H | R⁷ = H |
| R³ = H | | R⁴ = H | | | |
| Salz: 2HCl | | | | | |

### Beispiel 112

| | | | | | |
|---|---|---|---|---|---|
| X = O | R¹ = COiPr | R² = H | R⁵ = H | R⁶ = H | R⁷ = H |
| R³ = COMe | | R⁴ = 4-Acetylaminophenyl | | | |

### Beispiel 113

| | | | | | |
|---|---|---|---|---|---|
| X = O | R¹ = COiPr | R² = H | R⁵ = H | R⁶ = H | R⁷ = H |
| R³ = COMe | | R⁴ = Ph | | | |

### Messung der Hemmung der Aktivität gereinigter Stickstoffmonoxid-Synthase (NOS)

Bei diesem Aktivitätsassay wird das bei der Bildung von NO durch gereinigte NOS anfallende Koprodukt L-Citrullin quantitativ erfaßt. Als Substrat der Enzymreaktion wird ³H-radiomarkiertes L-Arginin eingesetzt, das zu ³H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillation gemessene ³H-Aktivität entspricht dann der Menge an L-Citrullin, die ein direktes Maß für die Aktivität der NOS ist.

Das Grundmedium für die Durchführung der Enzymreaktion ist TE-Puffer (Triethanolamin, EDTA, pH 7,0). Das Endvolumen jeder Inkubation beträgt 100 µl. Das Reaktionsgemisch wird erhalten, indem die folgenden 6 Komponenten auf Eis gemischt werden:
1. "REA-Mix" (pH 7,0), der Triethanolamin, Calciumchlorid, Magnesiumchlorid, EDTA, L-Arginin, Calmodulin und Flavin-Adenin-Dinukleotid (FAD) enthält;
2. frisch zubereitete Stammlösung von β-Nicotinamid-Adenin-Dinukleotid-Phosphat, reduzierte Form (NADPH);
3. (6R)-5,6,7,8-Tetrahydro-L-Biopterin-Dihydrochlorid-Stammlösung (BH₄) oder - für Versuche ohne BH₄ - stattdessen TE-Puffer;
4. gereinigte NO-Synthase aus Schweinekleinhirn oder aus Schweineleber;
5. L-[2,3,4,5-³H]-Arginin-Hydrochlorid-Stammlösung (1,5-2,6 TBq/mmol);
6. zu testende Substanz.

Die finalen Konzentrationen der Komponenten im Inkubationsvolumen von 100 µl sind:
Triethanolamin 50 mM, EDTA 0,5 mM, CaCl₂ 226 µM, MgCl₂ 477 µM, L-Arginin 50 µM, Calmodulin 0,5 µM, FAD 5 µM, NADPH 1 mM, BH₄ (wenn zugesetzt) 2 µM, zu testende Substanz 100 µM. Nach dem Mischen der Komponenten auf Eis wird der Reaktionsansatz sofort in einem Wasserbad bei 37°C für 15 Minuten inkubiert. Nach dieser Inkubationszeit wird die Reaktion durch Zugabe von 900 µl eiskaltem "Stoppuffer" (20 mM Natriumacetat, 2 mM EDTA, pH 5,5) abgestoppt und der Ansatz (Gesamtvolumen jetzt 1,0 ml) auf Eis gestellt. Zur Abtrennung des nicht umgesetzten ³H-L-Arginins wird das Gemisch auf eine Ionenaustauschersäule mit 0,8 ml Dowex AG 50 WX-8 (100-200 mesh) gegeben, die zuvor mit 2 ml Stoppuffer gespült und äquilibriert wurde. Nach dem Auftragen der Probe wird die Säule zweimal mit je 1 ml Wasser eluiert. Der Durchlauf der Probe und das Eluat werden in Szintillationsgefäßen aufgefangen und gereinigt (Gesamtvolumen 3 ml). Zu den 3 ml wäßriger Meßlösung werden 9 ml Szintillator-Lösung gegeben und die homogene Mischung wird in einem Flüssigszintillationszähler Tricarb 2500 TR (Packard) 1 Minute pro Probe gemessen. Die mit der zu testenden Substanz gefundene Aktivität wird in Prozent der Aktivität der Kontrolle angegeben. Jede Substanz wird in einer Konzentration von 100 µM in Anwesenheit von 2 µM Tetrahydrobiopterin auf antagonistische Wirkung sowie in Abwesenheit von Tetrahydrobiopterin auf agonistische Wirkung auf die NOS getestet. Alle Inkubationen werden in Triplikaten angesetzt. Jeder Versuch wird dreimal mit verschiedenen Enzympräparationen wiederholt. Einige Ergebnisse sind in der folgenden Tabelle angegeben.

| Verbindung des Beispiels | Enzym aus | Citrullin-Bildung (% der Kontrolle) |
|---|---|---|
| 57 | Schweinehirn | 66,3 |
| 69 | Schweinehirn | 52,3 |
| 70 | Schweinehirn | 42,5 |
| 71 | Schweinehirn | 46,2 |
| 73 | Schweinehirn | 0,0 |
| 74 | Schweinehirn | 42,6 |
| 80 | Schweinehirn | 1,5 |
| 82 | Schweinehirn | 6,7 |
| 85 | Schweinehirn | 0,0 |

Die nachfolgenden Beispiele betreffen pharmazeutische Zubereitungsformen

### Beispiel A

Gelatineweichkapseln, enthaltend 100 mg Wirkstoff pro Kapsel:

| | **pro Kapsel** |
|---|---|
| Wirkstoff | 100 mg |
| aus Kokosfett fraktioniertes Triclycerid-Gemisch | 400 mg |
| Kapselinhalt | 500 mg |

### Beispiel B

Injektionslösung, enthaltend 2,0 mg Wirkstoff pro ml:

| | **pro ml** |
|---|---|
| Wirkstoff | 2,0 mg |
| Polyethylenglycol 400 | 5,0 mg |
| Natriumchlorid | 2,7 mg |
| Wasser zu Injektionszwecken | ad 1 ml |

### Beispiel C

Emulsion, enthaltend 60 mg Wirkstoff pro 5 ml:

| | **pro 100 ml Emulsion** |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel D

Rektale Arzneiform, enthaltend 40 mg Wirkstoff pro Suppositorium:

| | **pro Suppositorium** |
|---|---|
| Wirkstoff | 40 mg |
| Suppositoriengrundmasse | ad 2 g |

### Beispiel E

Tabletten, enthaltend 40 mg Wirkstoff pro Tablette:

| | **pro Tablette** |
|---|---|
| Wirkstoff | 40 mg |
| Lactose | 600 mg |
| Maisstärke | 300 mg |
| lösliche Stärke | 20 mg |
| Magnesiumstearat | 40 mg |
| | 1000 mg |

### Beispiel F

Dragees, enthaltend 50 mg Wirkstoff pro Dragee:

| | **pro Dragee** |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel G

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

### Beispiel H

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

## Patentansprüche

1. Verwendung von Pteridin-Derivaten der allgemeinen Formel 1, 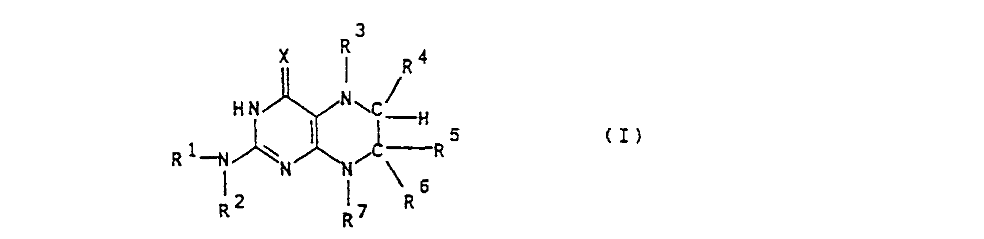 in der
X für O oder NH steht:
R¹ für Wasserstoff, Methyl, (C₁-C₅)-Alkanoyl, Nicotinoyl oder (1-Methvl-3-pyridinio)carbonyl steht:
R² für Wasserstoff oder Methyl steht:
R³ für Wasserstoff, Methyl, Ethyl, Benzyl, (C₁-C₅)-Alkanoyl, unsubstituiertes Benzoyl, substituiertes Benzoyl, Pyridoyl, Thienylcarbonyl, einen der Reste 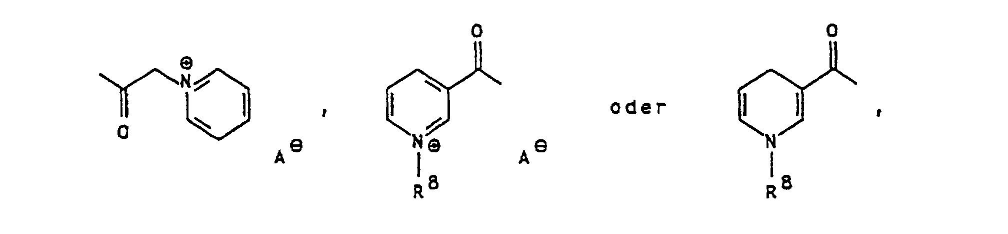 den Rest R⁹R^{9a}N-CO-, den Rest R⁹R^{9a}N-CS-, Phenoxycarbonyl oder Benzyloxycarbonyl steht:
R⁴ für Wasserstoff, (C₂-C₅)-Alkyl, unsubstituiertes Phenyl, substituiertes Phenyl oder den Rest R^{4a}-CH₂-steht;
R^{4a} für Wasserstoff, (C₁-C₄)-Alkylmercapto, den Rest -S(O)ₘR¹⁰, wobei m für die Zahlen 1 oder 2 steht, den Rest -NR¹¹R¹² oder den Rest -OR¹³ steht oder
R³ und R^{4a} zusammen für die Gruppierung -CO-O- stehen, wobei deren Carbonyl-C-Atom an die 5-Position des Pteridinmoleküls gebunden ist;
R⁵ für Wasserstoff, Methyl oder Phenyl steht;
R⁶ für Wasserstoff oder Methyl steht;
R⁷ für Wasserstoff oder Methyl steht:
R⁸ für (C₁-C₁₀)-Alkyl oder Benzyl steht;
R⁹ für Wasserstoff, (C₁-C₆)-Alkyl, Cyclohexyl, Phenyl oder Benzoyl steht;
R^{9a} für Wasserstoff, Methyl oder Ethyl steht;
R¹⁰ für Methyl steht;
R¹¹ und R¹² unabhängig voneinander für Wasserstoff oder Methyl stehen;
R¹³ für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
A^{Θ} für ein pharmakologisch verträgliches Anion steht;
oder ihrer tautomeren Formen oder ihrer pharmakologisch verträglichen Salze zur Herstellung eines Arzneimittels zur Vorbeugung oder Behandlung von Krankheiten, die durch einen erhöhten Stickstoffmonoxid-Spiegel bedingt sind.

2. Verwendung gemäß Anspruch 1, **dadurch gekennzeichnet, daß** X für Sauerstoff steht.

3. Verwendung gemäß Anspruch 1 und/oder 2, **dadurch gekennzeichnet, daß** R³ für Wasserstoff, (C₁-C₅)-Alkanoyl, unsubstituiertes Benzoyl, ein-, zwei- oder dreifach substituiertes Benzoyl, Thienylcarbonyl, Nicotinoyl, einen der Reste 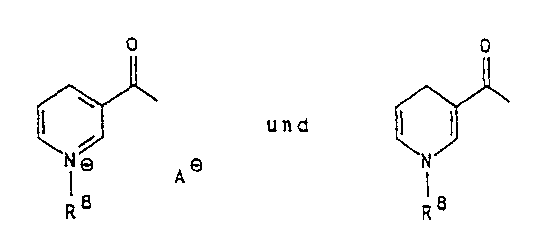 oder für R⁹NH-CO-, R⁹NH-CS- oder (CH₃)₂N-CO- steht.

4. Verwendung gemäß einem oder mehreren der Ansprüche 1 bis 3, **dadurch gekennzeichnet, daß** R⁴ für Wasserstoff, Phenyl, durch eine Acetylaminogruppe substituiertes Phenyl oder den Rest R^{4a}-CH₂- steht, wobei bevorzugt R^{4a} für Wasserstoff oder den Rest -OR¹³ steht, wobei besonders bevorzugt R¹³ für (C₁-C₁₀)-Alkyl oder (C₁-C₅)-Alkanoyl steht.

5. Verwendung gemäß einem oder mehreren der Ansprüche 1 bis 4 zur Herstellung eines Arzneimittels zur Behandlung oder Vorbeugung pathologischer Blutdruckabfälle, insbesondere beim septischen Schock oder der Krebstherapie mit Cytokinen.

6. Verwendung gemäß einem oder mehreren der Ansprüche 1 bis 4 zur Herstellung eines Arzneimittels zur Behandlung oder Vorbeugung von entzündlichen Erkrankungen, insbesondere Colitis ulcerosa.

7. Verwendung gemäß einem oder mehreren der Ansprüche 1 bis 4 zur Herstellung eines Arzneimittels zur Behandlung oder Vorbeugung von Infarktschäden und/oder Reperfusionsschäden.

8. Verwendung gemäß einem oder mehreren der Ansprüche 1 bis 4 zur Herstellung eines Arzneimittels zur Behandlung oder Vorbeugung von Transplantat-Abstoßungsreaktionen.

9. Verwendung gemäß einem oder mehreren der Ansprüche 1 bis 4 zur Herstellung eines Arzneimittels zur Behandlung oder Vorbeugung von Erkrankungen des Nervensystems aus der Reihe Morbus Alzheimer, Epilepsie und Migräne.

10. Pteridin-Derivate der allgemeinen Formel I, 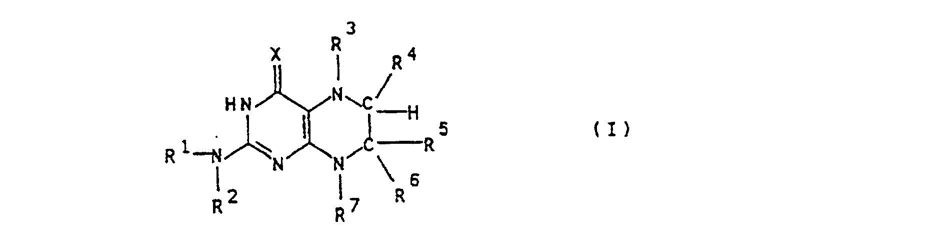 in der
X für O oder NH steht;
R¹ für Wasserstoff, Methyl, (C₁-C₅)-Alkanoyl, Nicotinoyl oder (1-Methyl-3-pyridinio)carbonyl steht;
R² für Wasserstoff oder Methyl steht;
R³ für Wasserstoff, Methyl, Ethyl, Benzyl, (C₁-C₅)-Alkanoyl, unsubstituiertes Benzoyl, substituiertes Benzoyl, Pyridoyl, Thienylcarbonyl, einen der Reste 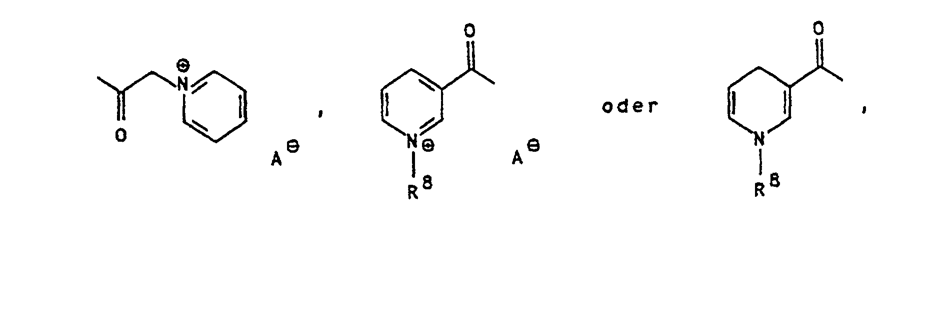 den Rest R⁹R^{9a}N-CO-, den Rest R⁹R^{9a}N-CS-, Phenoxycarbonyl oder Benzyloxycarbonyl steht;
R⁴ für Wasserstoff, (C₂-C₅)-Alkyl, unsubstituiertes Phenyl, substituiertes Phenyl oder den Rest R^{4a}-CH₂-steht;
R^{4a} für Wasserstoff, (C₁-C₄)-Alkylmercapto, den Rest -S(O)ₘR¹⁰, wobei m für die Zahlen 1 oder 2 steht, den Rest -NR¹¹R¹² oder den Rest -OR¹³ steht oder
R³ und R^{4a} zusammen für die Gruppierung -CO-O- stehen, wobei deren Carbonyl-C-Atom an die 5-Position des Pteridinmoleküls gebunden ist;
R⁵ für Wasserstoff, Methyl oder Phenyl steht;
R⁶ für Wasserstoff oder Methyl steht;
R⁷ für Wasserstoff oder Methyl steht;
R⁸ für (C₁-C₁₀)-Alkyl oder Benzyl steht;
R⁹ für Wasserstoff, (C₁-C₆)-Alkyl, Cyclohexyl, Phenyl oder Benzoyl steht;
R^{9a} für Wasserstoff, Methyl oder Ethyl steht;
R¹⁰ für Methyl steht;
R¹¹ und R¹² unabhängig voneinander für Wasserstoff oder Methyl stehen;
R¹³ für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
A^{Θ} für ein pharmakologisch verträgliches Anion steht;
oder ihre tautomeren Formen oder ihre pharmakologisch verträglichen Salze, wobei aber, wenn X für Sauerstoff und gleichzeitig R⁴ für den Rest R^{4a}-CH₂- steht, mindestens einer der Substituenten R¹, R², R³ und R⁷ eine andere Bedeutung als Wasserstoff hat, zur Verwendung als Arzneimittel.

11. Verfahren zur Herstellung eines Arzneimittels zur Vorbeugung oder Behandlung von Krankheiten, die durch einen erhöhten Stickstoffmonoxid-Spiegel bedingt sind, **dadurch gekennzeichnet, daß** als Wirkstoff mindestens ein Pteridin-Derivat der allgemeinen Formel I, 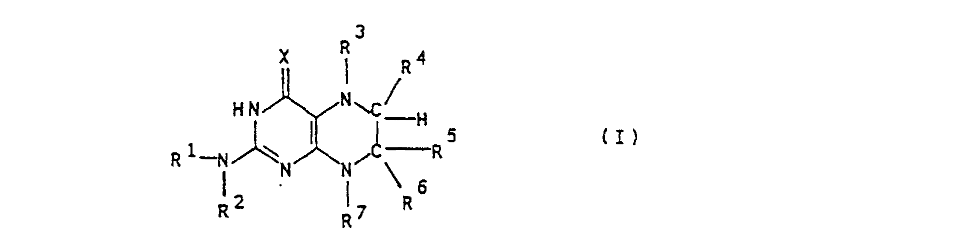 in der
X für O oder NH steht;
R¹ für Wasserstoff, Methyl, (C₁-C₅)-Alkanoyl, Nicotinoyl oder (1-Methyl-3-pyridinio)carbonyl steht;
R² für Wasserstoff oder Methyl steht;
R³ für Wasserstoff, Methyl, Ethyl, Benzyl, (C₁-C₅)-Alkanoyl, unsubstituiertes Benzoyl, substituiertes Benzoyl, Pyridoyl, Thienylcarbonyl, einen der Reste 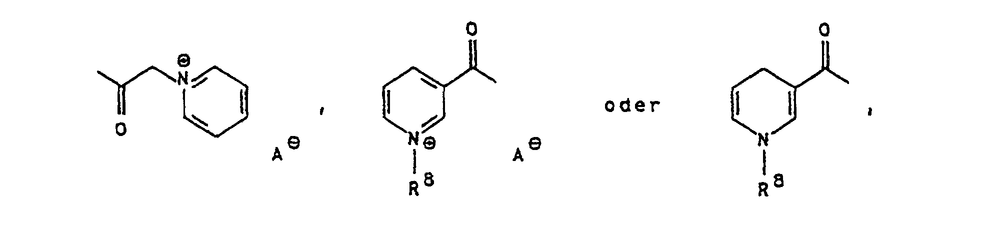 den Rest R⁹R^{9a}N-CO-, den Rest R⁹R^{9a}N-CS-, Phenoxycarbonyl oder Benzyloxycarbonyl steht;
R⁴ für Wasserstoff, (C₂-C₅)-Alkyl, unsubstituiertes Phenyl, substituiertes Phenyl oder den Rest R^{4a}-CH₂-steht;
R^{4a} für Wasserstoff, (C₁-C₄)-Alkylmercapto, den Rest -S(O)ₘR¹⁰, wobei m für die Zahlen 1 oder 2 steht, den Rest -NR¹¹R¹² oder den Rest -OR¹³ steht oder
R³ und R^{4a} zusammen für die Gruppierung -CO-O- stehen, wobei deren Carbonyl-C-Atom an die 5-Position des Pteridinmoleküls gebunden ist;
R⁵ für Wasserstoff, Methyl oder Phenyl steht;
R⁶ für Wasserstoff oder Methyl steht;
R⁷ für Wasserstoff oder Methyl steht:
R⁸ für (C₁-C₁₀)-Alkyl oder Benzyl steht;
R⁹ für Wasserstoff, (C₁-C₆)-Alkyl, Cyclohexyl, Phenyl oder Benzoyl steht;
R^{9a} für Wasserstoff, Methyl oder Ethyl steht;
R¹⁰ für Methyl steht;
R¹¹ und R¹² unabhängig voneinander für Wasserstoff oder Methyl stehen;
R¹³ für Wasserstoff, (C₁-C₁₀)-Alkyl, 2-Methoxyethyl, Phenyl, 3-Phenylpropyl, 3-Cyclohexylpropyl, (C₁-C₅)-Alkanoyl, Hydroxyacetyl, unsubstituiertes oder im Alkylteil durch einen Phenylrest substituiertes 2-Amino-(C₂-C₆)-alkanoyl oder ((C₁-C₂)-Alkoxy)carbonyl steht;
A^{Θ} für ein pharmakologisch verträgliches Anion steht;
oder eine tautomere Form oder ein pharmakologisch verträgliches Salz davon verwendet wird.

## Claims

1. The use of pteridine derivatives of the formula I 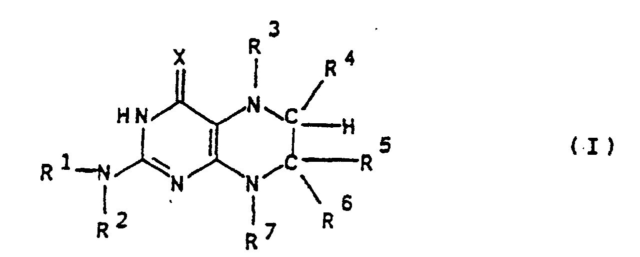 in which
X is O or NH;
R¹ is hydrogen, methyl, (C₁-C₅)-alkanoyl, nicotinoyl or (1-methyl-3-pyridinio)carbonyl;
R² is hydrogen or methyl;
R³ is hydrogen, methyl, ethyl, benzyl, (C₁-C₅)-alkanoyl, unsubstituted benzoyl, substituted benzoyl, pyridoyl, thienylcarbonyl, one of the radicals 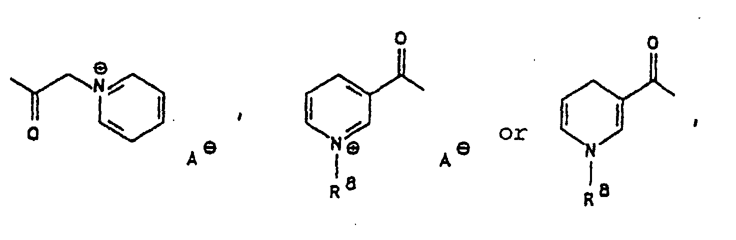 the radical R⁹R^{9a}N-CO-, the radical R⁹R^{9a}N-CS-, phenoxycarbonyl or benzyloxycarbonyl;
R⁴ is hydrogen, (C₂-C₅)-alkyl, unsubstituted phenyl, substituted phenyl or the radical R^{4a}-CH₂-;
R^{4a} is hydrogen, (C₁-C₄)-alkylmercapto, the radical -S(O)ₘR¹⁰, where m is the numbers 1 or 2, the radical -NR¹¹R¹² or the radical -OR¹³, or
R³ and R^{4a} together are the group -CO-O-, its carbonyl carbon atom being bonded to the 5-position of the pteridine molecule;
R⁵ is hydrogen, methyl or phenyl;
R⁶ is hydrogen or methyl:
R⁷ is hydrogen or methyl;
R⁸ is (C₁-C₁₀)-alkyl or benzyl;
R⁹ is hydrogen, (C₁-C₆)-alkyl, cyclohexyl, phenyl or benzoyl;
R^{9a} is hydrogen, methyl or ethyl;
R¹⁰ is methyl;
R¹¹ and R¹² independently of one another are hydrogen or methyl;
R¹³ is hydrogen, (C₁-C₁₀)-alkyl, 2-methoxyethyl, phenyl, 3-phenylpropyl, 3-cyclohexylpropyl, (C₁-C₅)-alkanoyl, hydroxyacetyl, 2-amino-(C₂-C₆)-alkanoyl which is unsubstituted or substituted in the alkyl moiety by a phenyl radical, or ((C₁-C₂)-alkoxy)carbonyl;
A^{θ} is a pharmacologically tolerable anion;
and their tautomeric forms and their pharmacologically tolerable salts for the preparation of a pharmaceutical for the prevention or treatment of diseases which are caused by an increased nitric oxide level.

2. The use as claimed in claim 1, wherein X is oxygen.

3. The use as claimed in claim 1 and/or 2, wherein R³ is hydrogen, (C₁-C₅)-alkanoyl, unsubstituted benzoyl, mono-, di- or trisubstituted benzoyl, thienylcarbonyl, nicotinoyl, one of the radicals 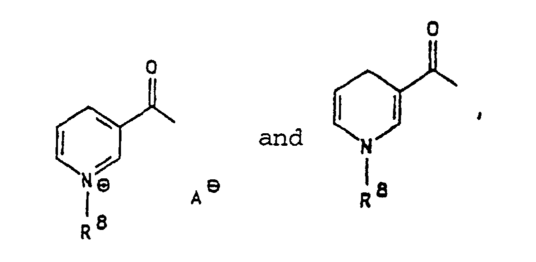 or R⁹NH-CO-, R⁹NH-CS- or (CH₃)₂N-CO-.

4. The use as claimed in one or more of claims 1 to 3, wherein R⁴ is hydrogen, phenyl, phenyl substituted by an acetylamino group or the radical R^{4a}-CH₂-, R^{4a} preferably being hydrogen or the radical -OR¹³, R¹³ particularly preferably being (C₁-C₁₀)-alkyl or (C₁-C₅)-alkanoyl.

5. The use as claimed in one or more of claims 1 to 4 for the preparation of a pharmaceutical for the treatment or prevention of pathological blood pressure decreases, in particular in septic shock or cancer therapy with cytokines.

6. The use as claimed in one or more of claims 1 to 4 for the preparation of a pharmaceutical for the treatment or prevention of inflammatory disorders, in particular ulcerative colitis.

7. The use as claimed in one or more of claims 1 to 4 for the preparation of a pharmaceutical for the treatment or prevention of infarct damage and/or reperfusion damage.

8. The use as claimed in one or more of claims 1 to 4 for the preparation of a pharmaceutical for the treatment or prevention of transplant rejection reactions.

9. The use as claimed in one or more of claims 1 to 4 for the preparation of a pharmaceutical for the treatment or prevention of disorders of the nervous system selected from the group consisting of Alzheimer's disease, epilepsy and migraine.

10. A pteridine derivative of the formula I 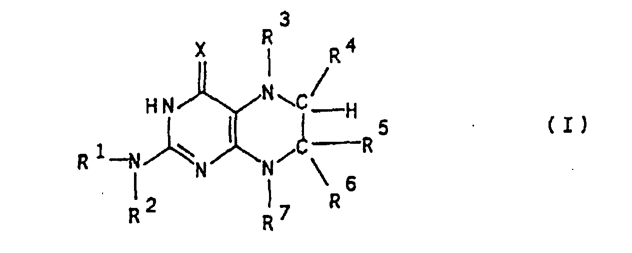 in which
X is O or NH;
R¹ is hydrogen, methyl, (C₁-C₅)-alkanoyl, nicotinoyl or (1-methyl-3-pyridinio)carbonyl;
R² is hydrogen or methyl;
R³ is hydrogen, methyl, ethyl, benzyl, (C₁-C₅)-alkanoyl, unsubstituted benzoyl, substituted benzoyl, pyridoyl, thienylcarbonyl, one of the radicals 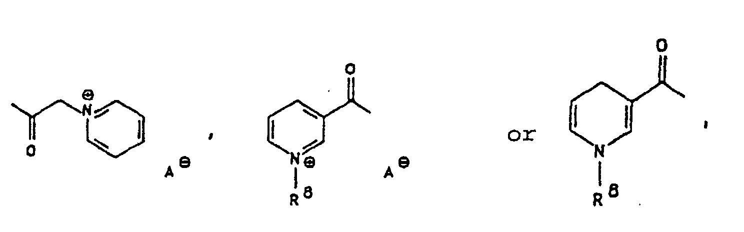 the radical R⁹R^{9a}N-CO-, the radical R⁹R^{9a}N-CS-, phenoxycarbonyl or benzyloxycarbonyl;
R⁴ is hydrogen, (C₂-C₅)-alkyl, unsubstituted phenyl, substituted phenyl or the radical R^{4a}-CH₂-;
R^{4a} is hydrogen, (C₁-C₄)-alkylmercapto, the radical -S(O)ₘR¹⁰, where m is the numbers 1 or 2, the radical -NR¹¹R¹² or the radical -OR¹³, or
R³ and R^{4a} together are the group -CO-O-, its carbonyl carbon atom being bonded to the 5-position of the pteridine molecule;
R⁵ is hydrogen, methyl or phenyl;
R⁶ is hydrogen or methyl;
R⁷ is hydrogen or methyl;
R⁸ is (C₁-C₁₀)-alkyl or benzyl;
R⁹ is hydrogen, (C₁-C₆)-alkyl, cyclohexyl, phenyl or benzoyl;
R^{9a} is hydrogen, methyl or ethyl;
R¹⁰ is methyl;
R¹¹ and R¹² independently of one another are hydrogen or methyl;
R¹³ is hydrogen, (C₁-C₁₀)-alkyl, 2-methoxyethyl, phenyl, 3-phenylpropyl, 3-cyclohexylpropyl, (C₁-C₅)-alkanoyl, hydroxyacetyl, 2-amino-(C₂-C₆)-alkanoyl which is unsubstituted or substituted in the alkyl moiety by a phenyl radical, or ((C₁-C₂)-alkoxy)carbonyl;
A^{σ} is a pharmacologically tolerable anion;
or their tautomeric forms or their pharmacologically tolerable salts where, however, if X is oxygen and at the same time R⁴ is the radical R^{4a}-CH₂-, at least one of the substituents R¹, R², R³ and R⁷ has a meaning other than hydrogen, for use as pharmaceuticals.

11. A process for preparing a pharmaceutical for the prevention or treatment of diseases which are caused by an increased nitric oxide level,
**characterized in that** as active compound at least one pteridine derivative is used of the formula I 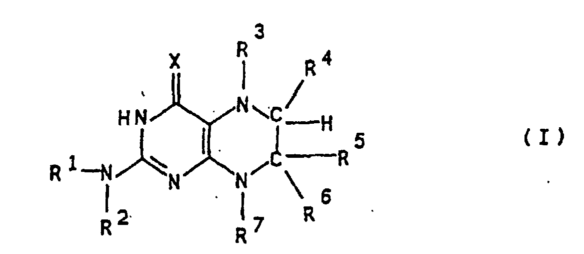 in which
X is O or NH;
R¹ is hydrogen, methyl, (C₁-C₅)-alkanoyl, nicotinoyl or (1-methyl-3-pyridinio)carbonyl;
R² is hydrogen or methyl;
R³ is hydrogen, methyl, ethyl, benzyl, (C₁-C₅)-alkanoyl, unsubstituted benzoyl, substituted benzoyl, pyridoyl, thienylcarbonyl, one of the radicals 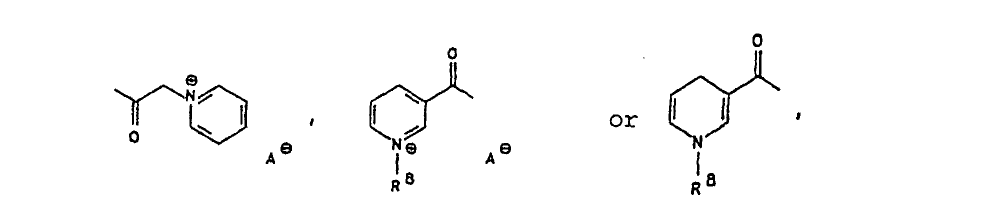 the radical R⁹R^{9a}N-CO-, the radical R⁹R^{9a}N-CS-, phenoxycarbonyl or benzyloxycarbonyl;
R⁴ is hydrogen, (C₂-C₅)-alkyl, unsubstituted phenyl, substituted phenyl or the radical R^{4a}-CH₂-;
R^{4a} is hydrogen, (C₁-C₄)-alkylmercapto, the radical -S(O)ₘR¹⁰, where m is the numbers 1 or 2, the radical -NR¹¹R¹² or the radical -OR¹³, or
R³ and R^{4a} together are the group -CO-O-, its carbonyl carbon atom being bonded to the 5-position of the pteridine molecule;
R⁵ is hydrogen, methyl or phenyl;
R⁶ is hydrogen or methyl;
R⁷ is hydrogen or methyl;
R⁸ is (C₁-C₁₀)-alkyl or benzyl;
R⁹ is hydrogen, (C₁-C₆)-alkyl, cyclohexyl, phenyl or benzoyl;
R^{9a} is hydrogen, methyl or ethyl;
R¹⁰ is methyl;
R¹¹ and R¹² independently of one another are hydrogen or methyl;
R¹³ is hydrogen, (C₁-C₁₀)-alkyl, 2-methoxyethyl, phenyl, 3-phenylpropyl, 3-cyclohexylpropyl, (C₁-C₅)-alkanoyl, hydroxyacetyl, 2-amino-(C₂-C₆)-alkanoyl which is unsubstituted or substituted in the alkyl moiety by a phenyl radical, or ((C₁-C₂)-alkoxy)carbonyl;
A^{σ} is a pharmacologically tolerable anion;
or a tautomeric form or a pharmacologically tolerable salt thereof.

## Revendications

1. Utilisation de dérivés de la ptéridine de formule générale I, 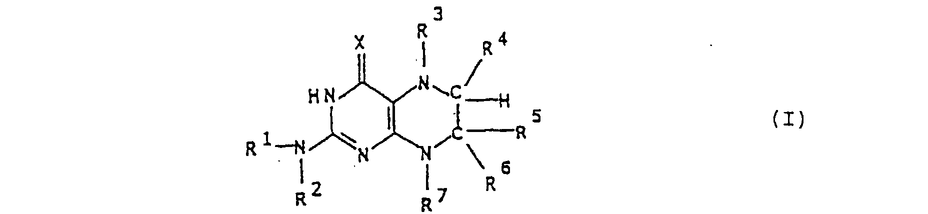 dans laquelle
X représente O ou NH ;
R¹ représente un atome d'hydrogène, des groupes méthyle, alcanoyle en C₁-C₅, nicotinoyle ou (1-méthyl-3-pyridinio)carbonyle ;
R² représente un atome d'hydrogène ou un groupe méthyle ;
R³ représente un atome d'hydrogène, des groupes méthyle, éthyle, benzyle, alcanoyle en C₁-C₅, benzoyle non substitué, benzoyle substitué, pyridoyle, thiénylcarbonyle, un des restes 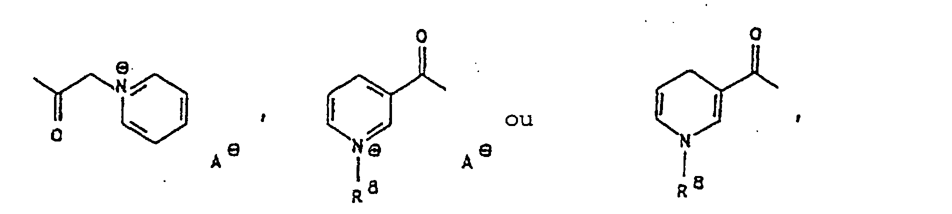 le reste R⁹R^{9a}N-CO-, le reste R⁹R^{9a}N-CS-, phénoxycarbonyle ou benzyloxycarbonyle ;
R⁴ représente un atome d'hydrogène, des groupes alkyle en C₂-C₅, phényle non substitué, phényle substitué ou le reste R^{4a}-CH₂- ;
R^{4a} représente un atome d'hydrogène, un groupe (alkyl en C₁-C₄)mercapto, le reste -S(O)ₘR¹⁰, m valant 1 ou 2, le reste -NR¹¹R¹² ou le reste -OR¹³ ou
R³ et R^{4a} ensemble représentent le groupement -CO-O-, leur atome de C carbonyle étant lié à la position 5 de la molécule de ptéridine ;
R⁵ représente un atome d'hydrogène, des groupes méthyle ou phényle ;
R⁶ représente un atome d'hydrogène ou un groupe méthyle ;
R⁷ représente un atome d'hydrogène ou un groupe méthyle ;
R⁸ représente un groupe alkyle en C₁-C₁₀ ou benzyle ;
R⁹ représente un atome d'hydrogène, des groupes alkyle en C₁-C₆, cyclohexyle, phényle ou benzoyle ;
R^{9a} représente un atome d'hydrogène, des groupes méthyle ou éthyle ;
R¹⁰ représente un groupe méthyle ;
R¹¹ et R¹² représentent, indépendamment l'un de l'autre, un atome d'hydrogène ou un groupe méthyle ;
R¹³ représente un atome d'hydrogène, des groupes alkyle en C₁-C₁₀, 2-méthoxyéthyle, phényle, 3-phénylpropyle, 3-cyclohexylpropyle, alcanoyle en C₁-C₅, hydroxyacétyle, un groupe 2-aminoalcanoyle en C₂-C₆ ou (alkoxy en C₁-C₂)carbonyle non substitué ou substitué dans le fragment alkyle par un reste phényle ;
A^{θ} représente un anion toléré du point de vue pharmacologique ; ou
leurs formes tautomères ou leurs sels tolérés du point de vue pharamcologique pour la préparation d'un médicament pour la prévention ou le traitement de maladies qui sont induites par un niveau élevé en monoxyde d'azote.

2. Utilisation selon la revendication 1, **caractérisée en ce** X représente un atome d'oxygène.

3. Utilisation selon la revendication 1 et/ou 2, **caractérisée en ce que** R³ représente un atome d'hydrogène, des groupes alcanoyle en C₁-C₅, benzoyle non substitué, benzoyle substitué une, deux ou trois fois, thiénylcarbonyle, nicotinoyle, un des restes 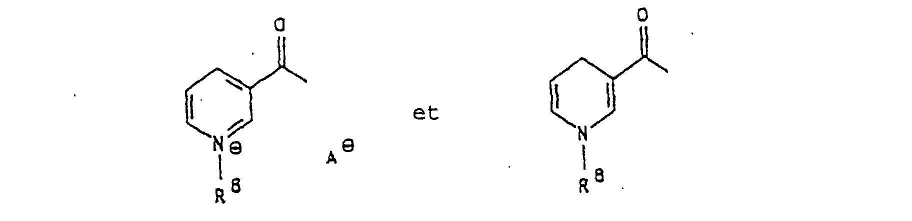 ou R⁹-NH-CO-, R⁹NH-CS- ou (CH₃)₂N-CO-.

4. Utilisation selon une ou plusieurs des revendications 1 à 3, **caractérisée en ce que** R⁴ représente un atome d'hydrogène, des groupes phényle, phényle substitué par un groupe acétylamino ou le reste R^{4a}-CH₂-, où R^{4a} représente de préférence un atome d'hydrogène ou le reste -OR¹³, de manière particulièrement préférée, R¹³ représentant de manière particulièrement préférée un groupe alkyle en C₁-C₁₀ ou alcanoyle en C₁-C₅.

5. Utilisation selon une ou plusieurs des revendications 1 à 4 pour la préparation d'un médicament pour le traitement ou la prévention de chutes de tension pathologiques, en particulier dans le choc septique ou dans la thérapie du cancer par des cytokines.

6. Utilisation selon une ou plusieurs des revendications 1 à 4, pour la préparation d'un médicament pour le traitement ou la prévention de maladies inflammatoires, en particulier de la colite ulcéreuse.

7. Utilisation selon une ou plusieurs des revendications 1 à 4 pour la préparation d'un médicament pour le traitement ou la prévention de dégâts provoqués par l'infarctus et/ou dégâts par reperfusion.

8. Utilisation selon une ou plusieurs des revendications 1 à 4 pour la préparation d'un médicament pour le traitement ou la prévention de réactions de rejets de greffes.

9. Utilisation selon une ou plusieurs des revendications 1 à 4 pour la préparation d'un médicament pour le traitement ou la prévention de maladies du système nerveux de type maladie d'Alzheimer, épilepsie et migraine.

10. Dérivés de la ptéridine de formule générale I 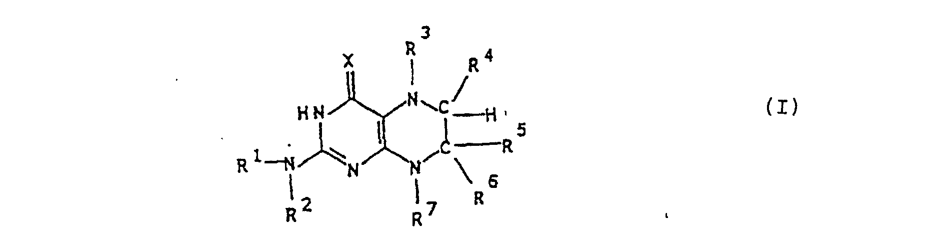 où
X représente O ou NH ;
R¹ représente un atome d'hydrogène, des groupes méthyle, alcanoyle en C₁-C₅, nicotinoyle ou (1-méthyl-3-pyridinio)carbonyle ;
R² représente un atome d'hydrogène ou un groupe méthyle ;
R³ représente un atome d'hydrogène, des groupes méthyle, éthyle, benzyle, alcanoyle en C₁-C₅, benzoyle non substitué, benzoyle substitué, pyridoyle, thiénylcarbonyle, un des restes 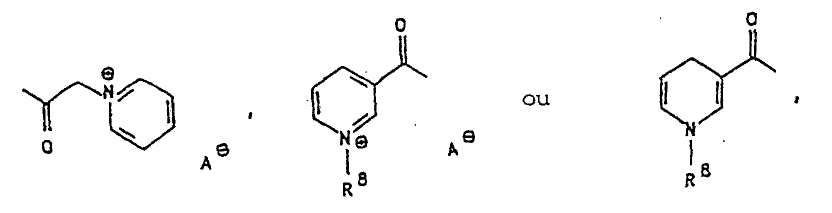 le reste R⁹R^{9a}N-CO-, le reste R⁹R^{9a}N-CS-, phénoxycarbonyle ou benzyloxycarbonyle ;
R⁴ représente un atome d'hydrogène, des groupes alkyle en C₂-C₅, phényle non substitué, phényle substitué ou le reste R^{4a}-CH₂- ;
R^{4a} représente un atome d'hydrogène, un groupe (alkyl en C₁-C₄)mercapto, le reste -S(O)ₘR¹⁰, m valant 1 ou 2, le reste -NR¹¹R¹² ou le reste -OR¹³ ou
R³ et R^{4a} ensemble représentent le groupement -CO-O-, leur atome de C carbonyle étant lié à la position 5 de la molécule de ptéridine :
R⁵ représente un atome d'hydrogène, des groupes méthyle ou phényle ;
R⁶ représente un atome d'hydrogène ou un groupe méthyle ;
R⁷ représente un atome d'hydrogène ou un groupe méthyle ;
R⁸ représente un groupe alkyle en C₁-C₁₀ ou benzyle :
R⁹ représente un atome d'hydrogène, des groupes alkyle en C₁-C₆, cyclohexyle, phényle ou benzoyle ;
R^{9a} représente un atome d'hydrogène, des groupes méthyle ou éthyle ;
R¹⁰ représente un groupe méthyle ;
R¹¹ et R¹² représentent, indépendamment l'un de l'autre, un atome d'hydrogène ou un groupe méthyle ;
R¹³ représente un atome d'hydrogène, des groupes alkyle en C₁-C₁₀, 2-méthoxyéthyle, phényle, 3-phénylpropyle, 3-cyclohexylpropyle, alcanoyle en C₁-C₅, hydroxyacétyle, un groupe 2-aminoalcanoyle en C₂-C₆ ou (alkoxy en C₁-C₂)carbonyle non substitué ou substitué dans le fragment alkyle par un reste phényle ;
A^{Θ} représente un anion toléré du point de vue pharmacologique ; ou
leurs formes tautomères ou leurs sels tolérés du point de vue pharmacologique, mais lorsque X représente un atome d'oxygène et R⁴ représente en même temps le reste R^{4a}-CH₂-, au moins un des substituants R¹, R², R³ et R⁷ posséde une signification différente de l'hydrogène, pour l'application en tant que médicament.

11. Procédé pour la préparation d'un médicament pour la prévention ou le traitement de maladies qui sont provoquées par un niveau élevé de monoxyde d'azote, **caractérisé en ce que** le principe actif est au moins un dérivé de la ptéridine de formule générale I 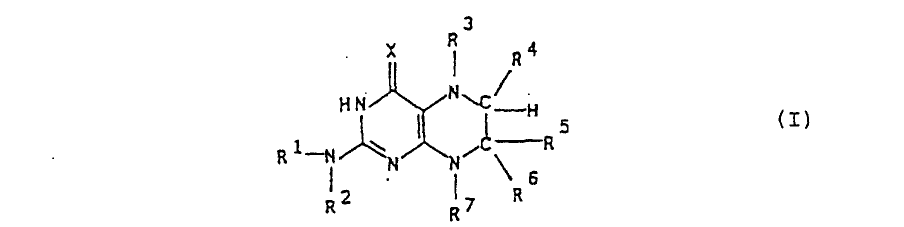
X représente O ou NH ;
R¹ représente un atome d'hydrogène, des groupes méthyle, alcanoyle en C₁-C₅, nicotinoyle ou (1-méthyl-3-pyridinio)carbonyle ;
R² représente un atome d'hydrogène ou un groupe méthyle ;
R³ représente un atome d'hydrogène, des groupes méthyle, éthyle, benzyle, alcanoyle en C₁-C₅, benzoyle non substitué, benzoyle substitué, pyridoyle, thiénylcarbonyle, un des restes 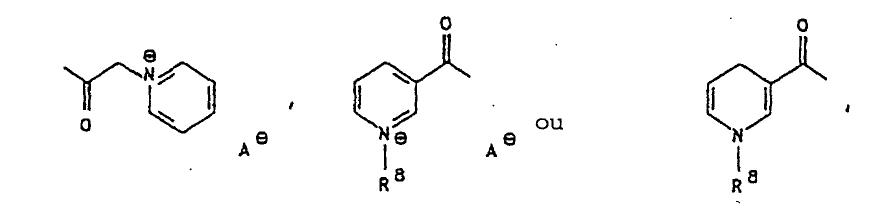 le reste R⁹R^{9a}N-CO-, le reste R⁹R^{9a}N-CS-, phénoxycarbonyle ou benzyloxycarbonyle ;
R⁴ représente un atome d'hydrogène, des groupes alkyle en C₂-C₅, phényle non substitué, phényle substitué ou le reste R^{4a}-CH₂- ;
R^{4a} représente un atome d'hydrogène, un groupe (alkyl en C₁-C₄)mercapto, le reste -S(O)ₘR¹⁰, m valant 1 ou 2, le reste -NR¹¹R¹² ou le reste -OR¹³ ou
R³ et R^{4a} ensemble représentent le groupement -CO-O-, leur atome de C carbonyle étant lié à la position 5 de la molécule de ptéridine ;
R⁵ représente un atome d'hydrogène, des groupes méthyle ou phényle ;
R⁶ représente un atome d'hydrogène ou un groupe méthyle ;
R⁷ représente un atome d'hydrogène ou un groupe méthyle ;
R⁸ représente un groupe alkyle en C₁-C₁₀ ou benzyle ;
R⁹ représente un atome d'hydrogène, des groupes alkyle en C₁-C₆, cyclohexyle, phényle ou benzoyle ;
R^{9a} représente un atome d'hydrogène, des groupes méthyle ou éthyle ;
R¹⁰ représente un groupe méthyle ;
R¹¹ et R¹² représentent, indépendamment l'un de l'autre, un atome d'hydrogène ou un groupe méthyle ;
R¹³ représente un atome d'hydrogène, des groupes alkyle en C₁-C₁₀, 2-méthoxyéthyle, phényle, 3-phénylpropyle, 3-cyclohexylpropyle, alcanoyle en C₁-C₅, hydroxyacétyle, un groupe 2-aminoalcanoyle en C₂-C₆ ou (alkoxy en C₁-C₂)carbonyle non substitué ou substitué dans le fragment alkyle par un reste phényle ;
A^{Θ} représente un anion toléré du point de vue pharmacologique ; ou
une forme tautomère ou un sel tolérés du point de vue pharmacologique.
